# EUROPÄISCHE PATENTSCHRIFT

(11) **EP 3 180 311 B1**
(45) Veröffentlichungstag und Bekanntmachung des Hinweises auf die Patenterteilung: **17.04.2019**
(21) Anmeldenummer: 15742007.6
(22) Anmeldetag: 23.07.2015
(51) Int. Cl.: B01J 31/02, B01J 31/22, C07C 403/24

(54) **VERFAHREN ZUR HERSTELLUNG VON ASTAXANTHIN AUS ASTACIN**
METHOD FOR PRODUCING ASTAXANTHIN FROM ASTACIN
PROCÉDÉ DE FABRICATION D'ASTAXANTHINE À PARTIR D'ASTACINE

(30) Priorität: 12.08.2014 EP 14180726
(43) Veröffentlichungstag der Anmeldung: 21.06.2017
(73) Patentinhaber: BASF SE, 67056 Ludwigshafen am Rhein (DE)
(72) Erfinder: SCHÄFER, Bernd, 76889 Dierbach (DE); SIEGEL, Wolfgang, 67117 Limburgerhof (DE)
(74) Vertreter: BASF IP Association
(86) Internationale Anmeldenummer: PCT/EP2015/066902
(87) Internationale Veröffentlichungsnummer: WO 2016/023732

(56) Entgegenhaltungen:
- US-A1- 2006 088 905
- ELIZABETH A. H. HALL ET AL: "Electrochemical reductive acylation of astacene; a route to the carotenoid astaxanthin", JOURNAL OF THE CHEMICAL SOCIETY, CHEMICAL COMMUNICATIONS, Nr. 9, 1. Januar 1978 (1978-01-01), Seite 387, XP055214977, ISSN: 0022-4936, DOI: 10.1039/c39780000387 in der Anmeldung erwähnt

## Beschreibung

Die vorliegende Erfindung betrifft ein Verfahren zur Herstellung von Astaxanthin aus Astacin. Ferner umfasst sie die Verwendung von Astacin zur Herstellung von Astaxanthin in unterschiedlichen isomeren Formen.
Die industriellen Synthesen des Astaxanthins sind sowohl in der einschlägigen Literatur, wie z. B. G. Britton, S. Liaaen-Jensen, H. Pfander, Carotenoids, Vol. 2, Birkhäuser Verlag, Basel, 1996, 283 ff., wie in verschiedenen Lehrbüchern, z. B. B. Schäfer, Naturstoffe der chemischen Industrie, Akademischer Verlag, Heidelberg, 2007, 427 ff., in wissenschaftlichen Zeitschriften, wie z. B. K. Meyer, Chemie in unserer Zeit 36 (2002) 178 sowie in der Patentliteratur, z. B. DE 10049271 A1 oder EP 1285912 A2 ausführlich beschrieben.

Obwohl von hohem technischen Interesse, ist die selektive Hydrierung der Doppelbindungen in Position Δ 2,3 resp. Δ 2',3' des Astacins zur Herstellung von Astaxanthin bisher völlig unbekannt. Lediglich in E. Widmer, T. Lukác, K. Bernhard, R. Zell, Helv. Chim. Acta 65 (1982) 671 findet man folgenden Hinweis:
"Astacin besitzt ein gewisses Interesse als potentielles Ausgangsmaterial für die Synthese von Astaxanthin. Zwar ist bisher kein Verfahren zur regioselektiven Hydrierung der 2,3-Doppelbindungen bekannt geworden. Jedoch gelang bereits eine elektrochemische Reduktion unter acylierenden Bedingungen, welche die Isolierung von Astaxanthin in einer Ausbeute von 10% erlaubte" [E. A. H. Hall, G. P. Moss, J. H. P. Utley, B. C. L. Weedon, Chem. Commun. (1978) 387 als der Erfindung naheliegendster Stand der Technik].

Allerdings sind Ausbeuten von 10 % bezogen auf die eingesetzte Menge an Astacin für industrielle Maßstäbe nicht zufriedenstellend. Außerdem fällt das Astaxanthin nach dem vorab genannten elektrolytischen Verfahren als Gemisch von Astaxanthin, Astacin und 2,3-Didehydroastaxanthin an. Solche komplexen Gemische lassen sich schwer in ihre Einzelbestandteile auftrennen. Reine oder hoch angereicherte Stereoisomere des Astaxanthins werden mit diesem Verfahren erst recht nicht erhalten.

Jedoch ist nicht nur die elektrochemische Synthese von Astaxanthin aus Astacin wenig zufriedenstellend. Auch die Herstellung dieses Moleküls unter Ramberg-Bäcklund-Bedingungen liefert keine hohen Astaxanthinausbeuten, sondern vielmehr große Mengen an Astacin, welches bis dato nicht wirtschaftlich in Astaxanthin weiter verarbeitet werden konnte. So liest man in Choi & Koo, J. Org. Chem. 70 (2005) auf S. 3330, rechte Spalte, im letzten Drittel des zweiten Absatzes: "The base-promoted dehydrosulfonation reaction of 11c using NaOEt in refluxing EtOH/benzene gave rise to astacene (3) in 71% yield instead of astaxanthin (2). Apparently, the silicon protecting groups were desilylated in the course of the harsh dehydrosulfonation reaction, and the resulting astaxanthin (2) was readily oxidized to astacene (3) in the presence of base and traces of oxygen.^{25"}

Widmer kommt in Helv. Chim. Acta. 65(3) 1982 671ff auf S. 678 im letzten Absatz i.V.m S. 679 Schema 10 zu dem Ergebnis, dass die Chemie an Carotinoiden mit einem C₄₀-Grundkörper schwierig ist. Dort heißt es: "Damit war einmal mehr demonstriert, dass chemische Reaktionen auf der C₄₀-Stufe fertig aufgebauter Carotinoide oft mit großen Problemen verbunden sein können, zumal auch die Reinigung der entstandenen Gemische schwierig wird."

Vor diesem Hintergrund besteht eine für den Fachmann zu lösende Aufgabe darin, die Probleme des Standes der Technik zu überwinden und ein Verfahren zu finden, um Astacin selektiv in Astaxanthin umzuwandeln. Selektiv bedeutet, neben den beiden Enolgruppen im Astacin- bzw. den entsprechenden Ketogruppen der tautomeren Form - vorhandene funktionelle Gruppen reagieren nicht oder nur in sehr geringem Umfang. Insbesondere soll eine Methode bereitgestellt werden, die Doppelbindungen in Position Δ^{2,3} respektive Δ^{2',3'} des Astacins regioselektiv zu Astaxanthin zu reduzieren. Nebenprodukte oder das Edukt Astacin selbst sollen kaum oder nur in kleinen Mengen entstehen. Das Verfahren soll mit geringem apparativem Aufwand möglich und in großtechnischem Maßstab umsetzbar sein. Es soll ferner kostengünstig, sowie einfach in der Durchführung sein, d.h. ohne viele Zwischenstufen oder aufwendige Verfahrensschritte bzw. Aufarbeitungsschritte ablaufen.

Eine weitere Aufgabe besteht darin, Astacin stereoselektiv umzusetzen, sprich so umzusetzen, dass zum größten Teil und nach Möglichkeit überhaupt nur jeweils ein Stereoisomer des Astaxanthins als Zielverbindung entsteht. Insbesondere soll eine Methode bereitgestellt werden, die Doppelbindungen in Position Δ^{2,3} respektive Δ^{2',3'} des Astacins stereoselektiv zu Astaxanthin zu reduzieren. Auch die stereoselektive Umsetzung soll leicht durchführbar und auf einen großtechnischen Maßstab übertragbar sein. Ferner soll die Umsetzung möglichst wenige Verfahrens- bzw. Aufarbeitungsschritte enthalten und kostengünstig sein.

Schließlich besteht eine Aufgabe darin, eine neue Vorstufe als Ausgangsmaterial für die Synthese von Astaxanthin aufzufinden, wobei das Asymmetriezentrum in Position 3, 3' des Astaxanthins wahlweise racemisch, oder (S)- oder (R)- konfiguriert ist.

Hauptmerkmale der erfinderischen Lösung dieser Aufgaben ergeben sich aus den Ansprüchen 1 und 15. Ausgestaltungen sind Gegenstand der Ansprüche 2 bis 14.

Gelöst werden vormals genannte Aufgaben mit einem Verfahren zur Herstellung von Astaxanthin der Formel 1, 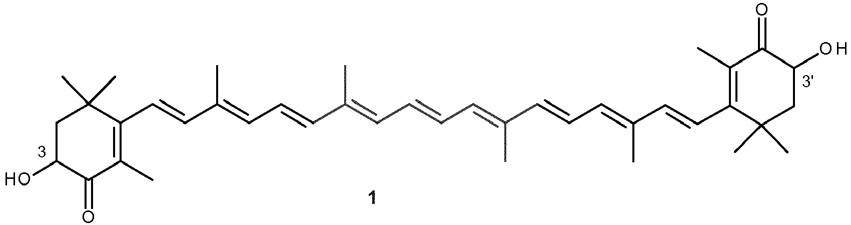 wobei das Asymmetriezentrum in Position 3 und 3' racemisch oder jeweils (S)- oder (R)- konfiguriert ist und die exozyklischen Doppelbindungen entweder E oder E und/oder Z konfiguriert sind. Dieses Verfahren zeichnet sich erfinderisch dadurch aus, dass Astacin der Formel 2, 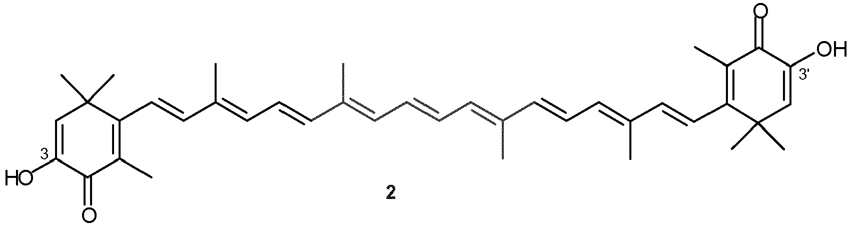 bei dem die exozyklischen Dopppelbindungen entweder E oder E und/oder Z konfiguriert sind, mit einem Reduktionsmittel stereounselektiv oder stereoselektiv umgesetzt wird, wobei das Reduktionsmittel wenigstens eine Verbindung ist, die ausgewählt ist aus der Gruppe bestehend aus einem sekundären Alkohol; Ameisensäure, den Salzen der Ameisensäure.

Ein solches Ergebnis war nicht vorherzusehen, denn neben dem enolisierten Carbonyl in Position 3 und/oder 3' würde der Fachmann davon ausgehen, dass wenigstens eine der weiteren funktionellen Gruppen der Verbindung 2, sprich das Carbonyl an Position 4 oder 4' und/oder wenigstens eine der exozyklischen Doppelbindungen ebenfalls reduziert würden. Der Fachmann würde erwarten, dass mit Nickel-Katalysatoren das nachfolgende Tetraol entsteht und mit Palladium-Katalysatoren Verbindungen des Typs A.

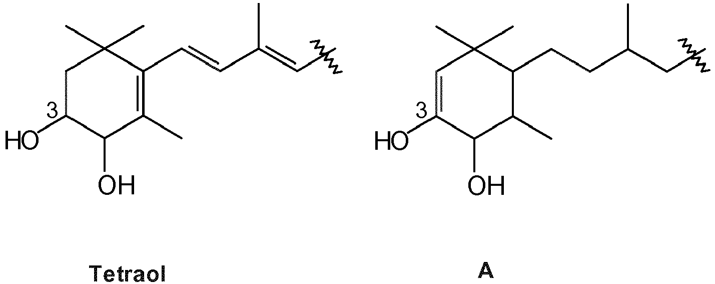

"Stereounselektiv" bedeutet, die durch das Reduktionsmittel hervorgebrachte Reaktion des Edukts führt zu einem Produkt ohne jegliche sterische Präferenz.

Unter "stereoselektiv" versteht man, das Reduktionsmittel bringt Produkte, nämlich Enantiomere bzw. Diastereomere hervor, die am Ort der Reduktion (am Stereozentrum) überwiegend nur eine Konfiguration ausbilden, nach Möglichkeit ausschließlich eine Konfiguration.

Das erfinderisch als Ausgangsverbindung eingesetzte Astacin 2 lässt sich auch als tautomeres Diketon gemäß dem nachfolgend gezeigten Gleichgewicht auffassen.

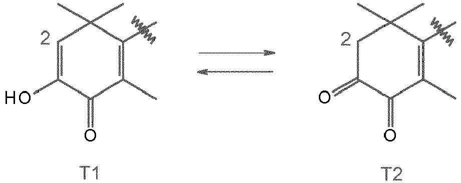

Deshalb schließt im Sinne dieser Erfindung der Begriff "Astacin" nicht nur das Tautomer T1 sondern jeweils immer auch das Tautomer T2 mit ein.

Zudem umfasst der Begriff "Astacin der Formel 2" oder "Astacin 2" nicht nur das all-trans-Isomer, wie oben als Struktur gezeichnet, sondern auch jegliches Z-Isomer, welches sich vom all-trans-Isomer ableitet sowie Mischungen aus wenigstens zweier dieser Isomere. "Z-Isomer" bedeutet, wenigstens eine der exozyklischen Doppelbindungen des Astacins 2 liegt in der Z-Konformation vor.

Unter "Astaxanthin der Formel 1" oder "Astaxanthin 1" versteht man neben dem all-trans-Isomer, wie oben als Struktur gezeichnet auch jegliches Z-Isomer, welches sich vom all-trans-Isomer ableitet sowie Mischungen aus wenigstens zweier dieser Isomere. "Z-Isomer" bedeutet, wenigstens eine der exozyklischen Doppelbindungen des Astaxanthins 1 liegt in der Z-Konformation vor.

Das Astaxanthin der Formel 1 wie im letzten Absatz definiert ist bezüglich seiner Stereochemie an Position 3, 3' aufteilbar in das "Astaxanthin der Formel 1a" 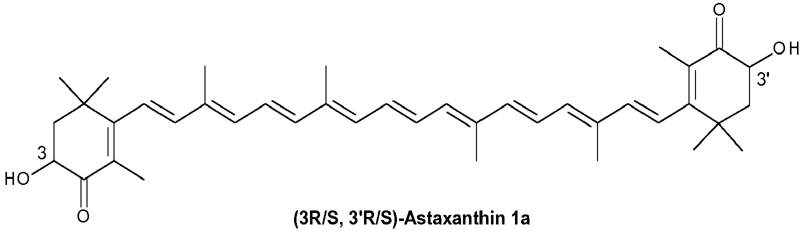 worunter jedes racemische Gemisch in Bezug auf die Positionen 3, 3' oder aber die Mesoform zu verstehen ist;
in das "Astaxanthin der Formel 1b" 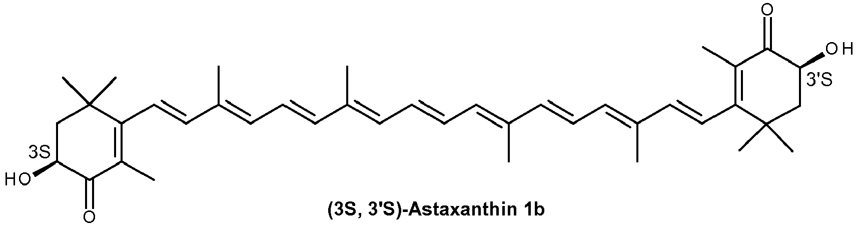 und in das "Astaxanthin der Formel 1c"

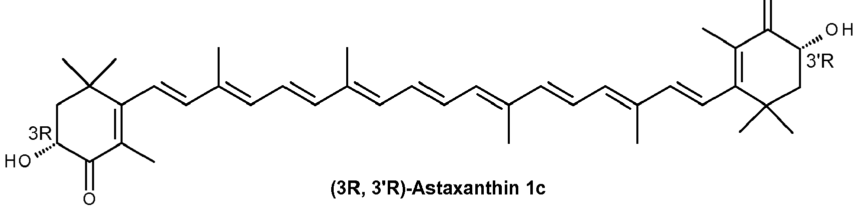

Auch die Formeln 1a, 1b und 1c des Astaxanthins umfassen jeweils das all-trans Isomere, jegliches Z-Isomere sowie Mischungen aus wenigstens zweier dieser Isomere, so wie vorab für die Begriffe "Astaxanthin der Formel 1" oder "Astaxanthin 1" erklärt.

Der Begriff Reduktionsmittel umfasst alle diejenigen Verbindungen, die geeignet sind, Astacin 2 in Astaxanthin 1 zu überführen. In einer bevorzugten Weiterführung sind unter dem Begriff Reduktionsmittel alle diejenigen Verbindungen zu verstehen, die Astacin 2 in Astaxanthin 1 überführen, ohne dass weitere funktionelle Gruppen des Astacins 2 mit umgesetzt werden.

Eine Fortführung der Erfindung sieht vor, dass das Reduktionsmittel wenigstens eine Verbindung ist, die ausgewählt ist aus der Gruppe bestehend aus Wasserstoffgas; einem sekundären Alkohol, vorzugsweise Isopropanol oder Butan-2-ol; Ameisensäure, den Salzen der Ameisensäure, insbesondere einem Alkali-, Erdalkali- oder Ammoniumformiat oder einem Mono-, Di-, Tri- oder Tetra(C1 - C4)-Alkylammoniumformiat.

Ein sekundärer Alkohol ist eine Verbindung, bei der sich am ipso-ständigen C-Atom zwei Alkylgruppen befinden, wobei Alkyl jede Gruppe mit der Summenformel CₙH₂ₙ₊₁ einschließt. Sekundäre Alkohole sind ausgewählt aus der Gruppe bestehend aus Propan-2-ol, Butan-2-ol, 3-Methylbutan-2-ol, 3,3-Dimethylbutan-2-ol, Pentan-2-ol, Pentan-3-ol, 2-Methylpentan-3-ol, 3-Methyl-pentan-2-ol, 4-Methylpentan-2-ol, 2,2-Dimethylpentan-3-ol, 2,4-Dimethylpentan-3-ol, 3,3-Di-methylpentan-2-ol, 4,4-Dimethylpentan-2-ol, 2,2,4-Trimethylpentan-3-ol, 2,2,4,4-Tetramethyl-pentan-3-ol, Hexan-2-ol, Hexan-3-ol, 2-Methylhexan-3-ol, 3-Methylhexan-2-ol, 4-Methylhexan-2-ol, 4-Methylhexan-3-ol, 4-Ethylhexan-3-ol, 5-Methylhexan-2-ol, 5-Methylhexan-3-ol, 2,2-Dimethylhexan-3-ol, 2,4-Dimethylhexan-3-ol, 2,5-Dimethylhexan-3-ol, 4-Isopropyl-2-methylhexan-3-ol, 2,2,5,5-Tetramethylhexan-3-ol, Heptan-2-ol, Heptan-3-ol, 2-Methylheptan-3-ol, 2-Methylheptan-4-ol, 3-Methylheptan-2-ol, 4-Methylheptan-2-ol, 4-Methylheptan-3-ol, 5-Methylheptan-2-ol, 5-Methylheptan-3-ol, 6-Methylheptan-2-ol, 2,2-Dimethylheptan-3-ol, 2,6-Dimethylheptan-3-ol, 2,6-Dimethylheptan-4-ol, 3,5-Dimethylheptan-4-ol, 3,6-Dimethylheptan-2-ol, 2,5,6-Trimethylheptan-4-ol, Octan-2-ol, Octan-3-ol, Octan-4-ol, 2-Methyloctan-4-ol, 2-Methyloctan-5-ol, 3-Methyloctan-2-ol, 3-Methyloctan-4-ol, 2,2-Dimethyloctan-3-ol, 2,4-Dimethyloctan-3-ol, 2,6-Dimethyloctan-3-ol, 3,7-Dimethyloctan-2-ol, Nonan-2-ol, Nonan-4-ol, Nonan-5-ol, Nonan-3-ol, 2-Methylnonan-4-ol, 2-Methylnonan-3-ol, 3-Methylnonan-2-ol, 5-Methylnonan-4-ol, 5-Ethylnonan-2-ol, 5-Butylnonan-2-ol, 2,2-Dimethylnonan-3-ol, 2,6,8-Trimethylnonan-4-ol, Decan-2-ol, Decan-3-ol, Decan-4-ol, Decan-5-ol, 2-Methyldecan-3-ol, 5-Methyldecan-4-ol, Undecan-2-ol, Undecan-3-ol, Undecan-5-ol, Undecan-6-ol, 2-Methylundecan-3-ol, 5-Methylundecan-6-ol, 6-Methylundecan-5-ol, 6-Pentylundecan-5-ol, 7-Ethyl-2-methylundecan-4-ol, Dodecan-2-ol, Dodecan-3-ol, Dodecan-5-ol, Dodecan-4-ol, Dodecan-6-ol, 2-Methyldodecan-3-ol, 3,7,11-Trimethyldodecan-4-ol, Tridecan-2-ol, Tridecan-3-ol, Tridecan-4-ol, Tridecan-7-ol, 2-Methyltridecan-3-ol, Tetradecan-2-ol, Tetradecan-3-ol, Tetradecan-4-ol, Tetradecan-6-ol, 2-Methyltetradecan-3-ol, 3,7-Dimethylpentadecan-2-ol, 6,10,14-Trimethylpentadecan-2-ol, Hexadecan-2-ol, Hexadecan-6-ol, 9-Octylheptadecan-10-ol, Dicaprylalkohol.

Unter diesen sekundären Alkoholen sind die Alkohole Isopropanol und/oder Butan-2-ol bevorzugt, da sie zum einen kostengünstig erhältlich sind und zum anderen bei der Reduktion Aceton oder Methylethylketon bilden, zwei Lösemittel, die sich aufgrund ihrer niedrigen Siedepunkte leicht abtrennen lassen. Zudem sorgen sie für eine partielle Löslichkeit der Verbindungen 1 und 2.

In einer weiteren Ausgestaltung ist das Reduktionsmittel bevorzugt wenigstens eine Verbindung, die ausgewählt ist aus der Gruppe Ameisensäure und/oder Salze der Ameisensäure. Diese Verbindungen sind preisgünstig in der Beschaffung. Zudem wird bei deren Reduktion Kohlendioxid frei, welches aus dem Reaktionsgefäß ohne große Mühen kontinuierlich oder aber am Ende der Reaktion freisetzbar ist. Hierdurch ist eine apparativ einfache und zielgerichtete Reaktionsführung möglich. Zudem vereinfacht es die Aufarbeitung der Reaktionsmischung erheblich und reduziert somit die Kosten des Herstellungsverfahrens.

Salze der Ameisensäure sind alle diejenigen Verbindungen, die ein Formiat Anion enthalten und als Gegenion ein organisches oder anorganisches Kation.

Ein Mono-, Di- Tri- oder Tetra(C1 - C4)-Alkylammoniumformiat enthält ein Formiat-Anion und ein Stickstoff tragendes Kation als Gegenion. Das Stickstoff tragende Kation ist ein Ammoniumion, welches neben Stickstoff vier Wasserstoffatome enthält oder anstelle von Wasserstoff eine (mono), zwei (di) drei (tri) oder vier (tetra) Alkylgruppen aufweist. Die wenigstens eine Alkylgruppe ist eine C1-C4-Alkylgruppe, d.h., sie ist ausgewählt aus methyl, ethyl, n-propyl, isopropyl, n-butyl, sek.-butyl, isobutyl, tert.-butyl.

Für die verfahrensgemäße Erfindung gut geeignete und wohlfeile Salze der Ameisensäure umfassen Trimethylammoniumformiat, Triethylammoniumformiat, Tri-n-butylammoniumformiat, Ethyldiisopropylammoniumformiat, Tetrabutylammoniumformiat oder eine Mischung aus wenigstens zweier dieser Salze.

Besonders bevorzugt ist das Reduktionsmittel ein Alkali- oder ein Erdalkalisalz oder ein Ammoniumsalz der Ameisensäure oder eine Mischung aus wenigstens zweier dieser Verbindungen. Neben dem gasförmigen Kohlendioxid fallen bei der Reduktion mit einem dieser Reduktionsmittel Nebenprodukte an, die entweder in einer polaren Phase gelöst oder aber als Salz ausgefallen vorliegen. Hierdurch fällt eine Abtrennung vom Reaktionsprodukt besonders leicht.

In einer besonders weitergebildeten bevorzugten Ausführungsform ist das Reduktionsmittel wenigstens eine Verbindung, die ausgewählt ist aus der Gruppe bestehend aus Natriumformiat, Kaliumformiat, Magnesiumformiat, Calciumformiat und unter den Ammoniumsalzen das des Ammoniaks, also Ammoniumformiat. Diese Reduktionsmittel haben gemein, dass sie sehr kostengünstig sind, weil die Formiate von Natrium, Kalium, Magnesium und Calcium, sowie die des Ammoniaks leicht zugänglich und von verschiedenen Anbietern zu erwerben sind. Ammoniumformiat bietet zudem den Vorteil, bei erhöhter Temperatur Ammoniak freizusetzen. So gesehen lässt sich mit Ende der Reduktion überschüssiges Ammoniumformiat durch Erhitzen zersetzen und abtrennen.

In einer weiteren Variante ist das Reduktionsmittel ausgewählt aus der Gruppe der Formiate primärer Amine, insbesondere aus dem Formiat wenigstens eines der Amine Methylamin, Ethylamin, n-Propylamin, Isopropylamin, n-Butylamin, sec.-Butylamin, tert.-Butylamin, Isobutylamin, n-Pentylamin, Anilin, Benzylamin.

In einer anderen Ausgestaltung der Erfindung ist das Reduktionsmittel wenigstens eine Verbindung, die ausgewählt ist aus der Gruppe bestehend aus sekundären oder tertiären Aminen der Ameisensäure. Sekundäre Amine der Ameisensäure sind aus einem Formiation als Anion und einem einfach protonierten N,N-Dialkylamin als Kation aufgebaut.

Sekundäre Amine der Ameisensäure umfassen das Formiat des Dimethylamins, des Diethylamins, des Di-n-Propylamins, des Di-n-Butylamins oder eine Mischung aus wenigstens zweier dieser Verbindungen.

Tertiäre Amine der Ameisensäure bestehen aus einem Formiation als Anion und einem einfach protonierten N,N,N-Trialkylamin als Kation.

Tertiäre Amine der Ameisensäure umfassen das Formiat des Trimethylamins, des Triethylamins, des Tri-n-propylamins, des Tri-n-butylamins, des Ethyldiisopropylamins oder eine Mischung aus wenigstens zweier dieser Verbindungen.

In einer noch anderen Abwandlung der Erfindung ist das Reduktionsmittel wenigstens eine Verbindung, die ausgewählt ist aus der Gruppe der quartären Ammoniumsalze der Ameisensäure. Quartäre Ammoniumsalze der Ameisensäure sind Verbindungen bestehend aus einem Formiation als Anion und einem N,N,N,N-Tetraalkylammonium Ion als Kation.

Quartäre Salze der Ameisensäure umfassen beispielsweise Tetraethylammoniumformiat, Tetrabutylammoniumformiat, Triisopropylethylammoniumformiat.

In einer weiteren Ausgestaltung ist das Reduktionsmittel bevorzugt wenigstens eine Verbindung, die ausgewählt ist aus der Gruppe der Salze der Ameisensäure, wobei die Salze durch Neutralisation von Ameisensäure mit einer entsprechenden Base *in situ* erzeugt werden. Diese Base ist ausgewählt aus der Gruppe Ammioniak und/oder primären Aminen und/oder sekundären Aminen und/oder tertiären Aminen. Derart generierte Salze der Ameisensäure sind immer dann besonders von Vorteil, wenn die Salzbildung nur langsam erfolgen soll oder wenn Salzverbindungen eingesetzt werden sollen, die nicht ohne weiteres käuflich zu erwerben sind.

In einer weiteren Ausführungsform ist das Reduktionsmittel bevorzugt wenigstens eine Verbindung, die ausgewählt ist aus der Gruppe der Salze der Ameisensäure, wobei die Salze als solche eingesetzt werden. Derartige Salze erweisen sich immer dann als günstig, wenn sie wohlfeil erhältlich und gut lagerbar sind und eine reaktionsabhängige Mindest- oder Maximalkonzentration dieser Salze nicht durch *in situ* Bildung reguliert werden muss.

Das erfinderische Verfahren wird dadurch weitergeführt, dass das Astacin 2 mit dem Reduktionsmittel in Gegenwart eines Übergangsmetallkatalysators, stereounselektiv oder stereoselektiv umgesetzt wird; bevorzugt in Gegenwart eines achiralen oder optisch aktiven Übergangsmetallkatalysators. Dies bedeutet, die weiter oben formulierte Aufgabe wurde in einer weiteren erfinderischen Ausführung durch eine übergangsmetallkatalysierte Hydrierung gelöst, wobei als Katalysatoren wahlweise achirale, enantiomerenangereicherte oder enantiomerenreine Übergangsmetallkatalysatoren eingesetzt werden. Diese Katalysatoren beschleunigen die erfinderische Reduktion deutlich und tragen somit zur Kostenreduktion bei.

Unter einem Übergangsmetallkatalysator versteht man eine Verbindung, die eine Reaktion beschleunigt. Sie enthält wenigstens ein Übergangsmetall, d.h. wenigstens ein Metall der dritten bis zwölften Gruppe des Periodensystems und wenigstens einen Liganden.

Ein optisch aktiver Übergangsmetallkatalysator ist ebenfalls eine Verbindung, die eine Reaktion beschleunigt. Sie enthält wenigstens ein Übergangsmetall der dritten bis zwölften Gruppe des Periodensystems sowie wenigstens einen optisch aktiven Liganden.

Optisch aktive Liganden sind solche Liganden, die in der Lage sind, die Polarisationsebene eines Strahls linear polarisierten Lichts mehr oder weniger zu drehen.

Es hat sich gezeigt, dass der Übergangsmetallkatalysator mit einem Übergangsmetall, das ausgewählt ist aus der Gruppe bestehend aus Ti, Zr, Hf, V, Nb, Ta, Cr, Mo, W, Mn, Re, Fe, Ru, Os, Co, Rh, Ir, Ni, Pd, Pt, Cu, Ag und Au für das erfinderische Verfahren heranzuziehen ist. Die Übergangsmetalle Zr, Nb, Mo, W, Ru, Co, Rh, Ir, Ni, Pd sind aufgrund ihrer relativen Verfügbarkeit und/oder ihrer Reaktionsfreudigkeit besonders geeignet, insbesondere die Übergangsmetalle Mo, Ru, Co, Rh, Ir, Ni, Pd. Besonders gute Ergebnisse wurden mit den Übergangsmetallen Ru, Ir, Ni, Pd bei entsprechender Ligandenanordnung erhalten. In den durchgeführten Versuchen schließlich hat sich Ruthenium (Ru) als besonders geeignet für das erfinderische Verfahren erwiesen, denn es konnten hohe Ausbeuten an Astaxanthin 1 erhalten werden, ohne dass dabei die Carbonylgruppe an Position 4 sowie weitere funktionelle Gruppen des Astacins 2 merklich mit umgesetzt wurden, nach Möglichkeit überhaupt nicht mit umgesetzt wurden.

Bevorzugt enthält der Übergangsmetallkatalysator, der zur Reduktion der Doppelbindung an der Position Δ^{2,3} (äquivalent zu einer Ketogruppe in Position 3) des Astacins 2 zum entsprechenden sekundären Alkohol geeignet ist, ein Übergangsmetallatom und mindestens einen wahlweise achiralen oder optisch aktiven Liganden. Als Übergangsmetallatom kommen prinzipiell alle Übergangsmetalle, wie beispielsweise Ti, Zr, Hf, V, Nb, Ta, Cr, Mo, W, Mn, Re, Fe, Ru, Os, Co, Rh, Ir, Ni, Pd, Pt, Cu, Ag oder Au in Frage, die einen geeigneten Übergangsmetallkatalysator bilden können.

Eine Weiterleitung des erfinderischen Verfahrens sieht vor, dass der Übergangsmetallkatalysator wenigstens einen Liganden enthält, der ausgewählt ist aus Aminen und/oder Phosphanen. Insbesondere ist der Ligand ausgewählt aus Aminen und/oder Phosphanen und/oder aromatischen Verbindungen und/oder Halogeniden. Dabei sind die aromatischen Verbindungen komplex an das Übergangsmetall gebunden und optional kovalent an einen Amin- oder Phosphanliganden. Derartige Spezies haben sich nämlich als besonders geeignet erwiesen, wenn es darum geht, mit einem Übergangsmetall zu koordinieren.

Insbesondere wurde festgestellt, dass das Astacin 2 dann besonders mit dem Reduktionsmittel in Gegenwart eines Übergangsmetallkatalysators stereounselektiv oder stereoselektiv umgesetzt wird; bevorzugt in Gegenwart eines optisch aktiven Übergangsmetallkatalysators, wenn das Übergangsmetall des Übergangsmetallkatalysators Ruthenium (Ru) ist und der Ligand aus Aminen ausgewählt ist.

Der Ligand Phosphan ist bevorzugt ein Phosphan der allgemeinen Formel 3, 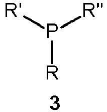 wobei R, R' und R" unabhängig voneinander ausgewählt sind aus der Gruppe bestehend aus einem der Reste C1-C4-Alkyl, Phenyl, ein bis dreifach C1-C4-Alkyl-substituiertes Aryl; bevorzugt ein Triarylphosphan und ganz besonders bevorzugt Triphenylphosphan.

C1-C4-Alkyl bedeutet eine Gruppe, die ausgewählt ist aus methyl, ethyl, n-propyl, isopropyl, n-butyl, sek.-butyl, isobutyl, tert.-butyl. Aryl umfasst alle aromatischen Grundkörper, insbesondere Phenyl und Benzyl. C1-C4-Alkyl-substituiertes Aryl steht für ein Aryl, wie vorher definiert, das mit einem, zwei, drei, vier oder fünf C1-C4-Alkylresten, wie vorher definiert, verknüpft ist.

Einen herausragenden Aspekt stellt folgende Ausgestaltung des erfinderischen Verfahrens unter Schutz. Sie beansprucht, dass der Übergangsmetallkatalysator wenigstens einen Liganden enthält, der ausgewählt ist aus der Gruppe bestehend aus H₂N-CH₂-CH₂-OH, MeHN-CH₂-CH₂-OH, H₂N-CH₂-CH₂-NH₂, TsNH-CH₂-CH₂-NH₂, TsNH-CH₂-CH₂-NH-(CH₂)ₙ-Oₘ-(CH₂)ₒ-Aryl mit n = 1 - 4, m = 0 oder 1 und o = 1 - 4 und Aryl = Phenyl oder Mono-, Di-, Tri-C1 - C4-Alkylphenyl, optisch aktive Verbindung. Es konnte nämlich mit diesem Typ von Liganden racemisches Astaxanthin 1a in Ausbeuten von 63,7 % erhalten werden, wie in Beispiel 2 gezeigt.

Deshalb sieht eine besondere Weiterleitung der Erfindung für ein Verfahren zur Herstellung von Astaxanthin der Formel 1 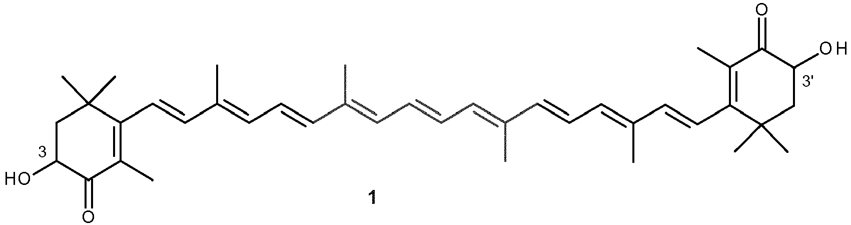 wobei das Asymmetriezentrum in Position 3 und 3' racemisch, oder (S)- oder (R)- konfiguriert ist und die exozyklischen Dopppelbindungen entweder E oder E und/oder Z konfiguriert sind, vor, dass das Astacin der Formel 2, 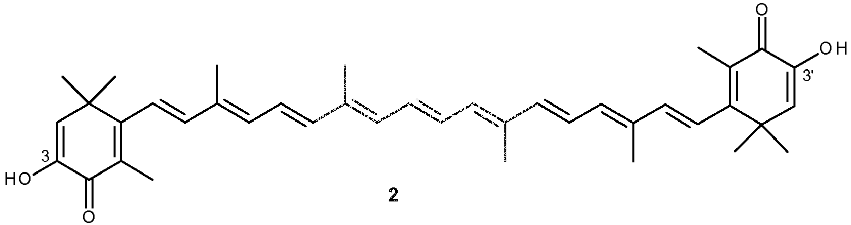 bei dem die exozyklischen Dopppelbindungen entweder E oder E und/oder Z konfiguriert sind, mit einem Reduktionsmittel, das ausgewählt ist aus der Gruppe bestehend aus Ameisensäure und/oder den Salzen der Ameisensäure, Isopropanol, Butan-2-ol, in Gegenwart eines Übergangsmetallkatalysators stereounselektiv oder stereoselektiv umgesetzt wird, wobei der Übergangsmetallkatalysator als Übergangsmetall Ruthenium (Ru) enthält und wenigstens einen Liganden, der ausgewählt ist aus der Gruppe bestehend aus H₂N-CH₂-CH₂-OH, MeHN-CH₂-CH₂-OH, H₂N-CH₂-CH₂-NH₂, TsNH-CH₂-CH₂-NH₂, TsNH-CH₂-CH₂-NH-(CH₂)ₙ-Oₘ-(CH₂)ₒ-Aryl mit n = 1 - 4, m = 0 oder 1 und o = 1 - 4 und Aryl = Phenyl oder Mono-, Di-, Tri-C1 - C4-Alkylphenyl, optisch aktive Verbindung.

Nach diesem erfindungsgemäßen Verfahren, welches eine stereounselektive und im Falle der optisch aktiven Verbindung entweder eine stereounselektive oder eine stereoselektive Reduktion beschreibt, stellt man racemische Gemische von Astaxanthin 1 her, die für viele Anwendungen hinreichend sind.

Die für die racemischen Gemische erforderlichen Übergangsmetallkatalysatoren lassen sich beispielsweise durch Umsetzung einer geeigneten Rutheniumverbindung wie beispielsweise [RuX₂(η⁶-Ar)]₂ mit einem geeigneten Liganden erzeugen, wobei X ein Halogenatom wie Fluor, Chlor, Brom oder Jod bedeutet, und Ar für Benzol oder ein substituiertes Benzolderivat, insbesondere ein mit C1-C4-Alkylresten substituiertes Benzolderivat steht. C1-C4-Alkyl besitzt die weiter oben bereits angegebene Bedeutung.

Jedoch besteht neben optisch inaktivem Astaxanthin auch Bedarf an optisch aktivem Astaxanthin.

Besonders bevorzugt wird deshalb in dem erfindungsgemäßen Verfahren ein optisch aktiver Übergangsmetallkatalysator eingesetzt, der ein Übergangsmetallatom und mindestens einen optisch aktiven, Liganden enthält, wobei das Übergangsmetallatom Ruthenium (Ru) ist. Dies bedeutet, dass das Astacin 2 mit dem Reduktionsmittel in Gegenwart eines optisch aktiven Übergangsmetallkatalysators, stereoselektiv umgesetzt wird, wobei der Übergangsmetallkatalysator ein Übergangsmetallatom und mindestens einen optisch aktiven Liganden enthält und das Übergangsmetallatom Ruthenium (Ru) ist.

Bevorzugte chirale, insbesondere optisch aktive Rutheniumkatalysatoren lassen sich beispielsweise durch Umsetzung einer geeigneten Rutheniumverbindung wie beispielsweise [RuX₂(η⁶-Ar)]₂ mit einem geeigneten chiralen, insbesondere optisch aktiven Liganden erzeugen, wobei X ein Halogenatom wie Fluor, Chlor, Brom oder Jod bedeutet, und Ar für Benzol oder ein substituiertes Benzolderivat, insbesondere ein mit C1-C4-Alkylresten substituiertes Benzolderivat steht. C1-C4-Alkyl besitzt die weiter oben bereits angegebene Bedeutung.

Der chirale, insbesondere optisch aktive Rutheniumkatalysator zeichnet sich bevorzugt dadurch aus, dass der optisch aktive Ligand ein optisch aktives Amin oder eine optisch aktive Aminosäure ist. Beispiele für optisch aktive Amine, die mit einer geeigneten Rutheniumverbindung, insbesondere [RuX₂(η⁶-Ar)]₂ zum katalytisch aktiven Komplex umgesetzt werden können, sind H₂N-CHPh-CHPh-OH, H₂N-CHMe-CHPh-OH, MeHN-CHMe-CHPh-OH, TsNH-CHPh-CHPh-NH₂, (1S,2S)-N-p-Touluolsulfonyl-1 ,2-diphenylethylendiamin, (1R,2R)- N-p-Touluolsulfonyl-1 ,2-diphenylethylendiamin, N-[(1S,2S)-1,2-Diphenyl-2-(2-(4-methylbenzyloxy)ethylamino)-ethyl]-4-methylbenzol sulfonamid oder N-[(1R,2R)-1,2-Diphenyl-2-(2-(4-methylbenzyloxy)ethylamino)-ethyl]-4-methylbenzol sulfonamid insbesondere (1S,2S)-N-p-Toluolsulfonyl-1,2-diphenylethylendiamin, (1R,2R)-N-p-Toluolsulfonyl-1,2-diphenylethylendiamin, N-[(1S,2S)-1,2-Diphenyl-2-(2-(4-methylbenzyloxy)ethylamino)-ethyl]-4-methylbenzol sulfonamid oder N-[(1R,2R)-1,2-Diphenyl-2-(2-(4-methylbenzyloxy)ethylamino)-ethyl]-4-methylbenzol sulfonamid.

Mithin sieht eine weitere wichtige erfinderische Ausgestaltung vor, dass der Übergangsmetallkatalysator wenigstens einen Liganden enthält, der ausgewählt ist aus der Gruppe bestehend aus einem optisch aktiven Amin, insbesondere H₂N-CHPh-CHPh-OH, H₂N-CHMe-CHPh-OH, MeHN-CHMe-CHPh-OH, TsNH-CHPh-CHPh-NH₂, (1S,2S)-N-p-Touluolsulfonyl-1,2-diphenylethylendiamin, (1R,2R)- N-p-Touluolsulfonyl-1,2-diphenylethylendiamin, N-[(1S,2S)-1,2-Diphenyl-2-(2-(4-methylbenzyloxy)ethylamino)-ethyl]-4-methylbenzol sulfonamid, N-[(1R,2R)-1,2-Diphenyl-2-(2-(4-methylbenzyloxy)ethylamino)-ethyl]-4-methylbenzol sulfonamid, einer optisch aktiven Aminosäure; und der bevorzugt ausgewählt ist aus der Gruppe bestehend aus H₂N-CHPh-CHPh-OH, H₂N-CHMe-CHPh-OH, MeHN-CHMe-CHPh-OH, TsNH-CHPh-CHPh-NH₂, (1S,2S)-N-p-Touluolsulfonyl-1,2- diphenylethylendiamin, (1R,2R)- N-p-Touluolsulfonyl-1,2-diphenylethylendiamin, N-[(1S,2S)-1,2-Diphenyl-2-(2-(4-methylbenzyloxy)ethylamino)-ethyl]-4-methylbenzol sulfonamid, N-[(1R,2R)-1,2-Diphenyl-2-(2-(4-methylbenzyloxy)ethylamino)-ethyl]-4-methylbenzol sulfonamid. Die bevorzugten Liganden zeichnen sich dadurch aus, dass sie besonders bereitwillig einen Komplex mit dem Übergangsmetall bilden. Dieser Übergangsmetallkomplex ist unter den Bedingungen des erfinderischen Verfahrens sehr stabil.

Enthält der Übergangsmetallkatalysator nach der Erfindung die im letzten Absatz genannten Liganden, wird eine einfache und effiziente Route eingeschlagen, stark stereoisomerenangereichertes oder gar stereoisomerenreines Astaxanthin 1 in guten Ausbeuten aus Astacin 2 herzustellen, wie weiter unten gezeigt.

Deshalb ist auch eine besonders wichtige Weiterleitung erfindungswesentlich. Sie umfasst ein Verfahren zur Herstellung von Astaxanthin der Formel 1 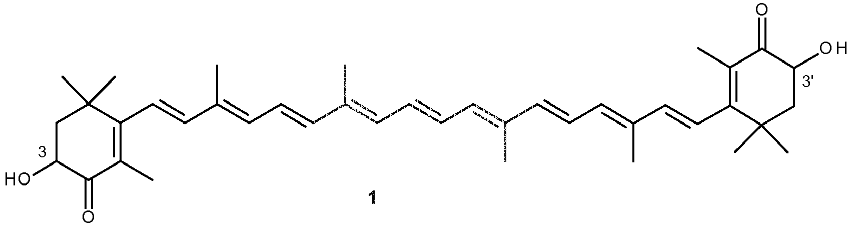 wobei das Asymmetriezentrum in Position 3 und 3' (S)- oder (R)- konfiguriert ist und die exozyklischen Dopppelbindungen entweder E oder E und/oder Z konfiguriert sind, bei dem man erfindungsgemäß Astacin der Formel 2, 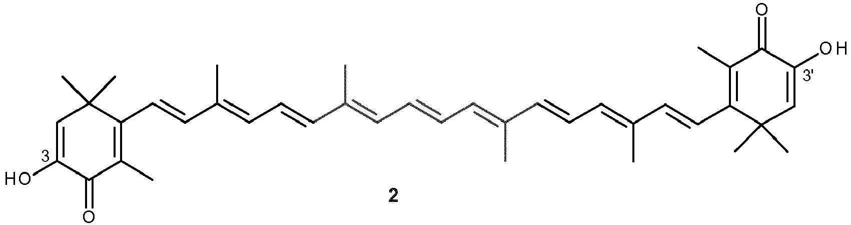 bei dem die exozyklischen Dopppelbindungen entweder E oder E und/oder Z konfiguriert sind, mit einem Reduktionsmittel, das ausgewählt ist aus der Gruppe bestehend aus Ameisensäure, den Salzen der Ameisensäure, Isopropanol oder Butan-2-ol, in Gegenwart eines optisch aktiven, vorzugsweise eines enantiomerenreinen Übergangsmetallkatalysators stereoselektiv umsetzt, wobei der optisch aktive, vorzugsweise der enantiomerenreine Übergangsmetallkatalysator als Übergangsmetall Ruthenium (Ru) enthält und wenigstens einen Liganden, der ausgewählt ist aus der Gruppe bestehend aus H₂N-CHPh-CHPh-OH, H₂N-CHMe-CHPh-OH, MeHN-CHMe-CHPh-OH, TsNH-CHPh-CHPh-NH₂, (1S,2S)-N-p-Touluolsulfonyl-1,2-diphenylethylendiamin, (1R,2R)- N-p-Touluolsulfonyl-1,2-diphenylethylendiamin, N-[(1S,2S)-1,2-Diphenyl-2-(2-(4-methylbenzyloxy)ethylamino)-ethyl]-4-methylbenzol sulfonamid, N-[(1R,2R)-1,2-Diphenyl-2-(2-(4-methylbenzyloxy)ethylamino)-ethyl]-4-methylbenzol sulfonamid, einer optisch aktiven Aminosäure.

Die jeweils anderen Stereoisomeren, sprich das jeweils andere Enantiomer oder das Diastereomere des Astaxanthins 1, fallen bei diesem Verfahren nur in sehr geringen Mengen und bevorzugt überhaupt nicht an.

Wird beispielsweise in dem erfindungsgemäßen Verfahren (1S,2S)-N-p-Toluolsulfonyl-1,2-diphenylethylendiamin als optisch aktiver Ligand eingesetzt, so erhält man in hoher Enantiomerenreinheit die Verbindung der Formel (3S, 3'S-1b), während man bei Einsatz von (1R,2R)-N-p-Toluolsulfonyl-1,2-diphenylethylendiamin als optisch aktivem Ligand die Verbindung der Formel (3R, 3'R-1c) erzeugt.

Es hat sich beim erfindungsgemäßen Verfahren herausgestellt, dass die meisten der Übergangsmetallkatalysatoren inklusive der meisten der chiralen Übergangsmetallkatalysatoren besonders effizient sind, wenn wenigstens einer ihrer Liganden einfach deprotoniert ist.

Besonders bevorzugt ist auch ein chiraler Rutheniumkatalysator, worin der optisch aktive Ligand durch einfache Deprotonierung von H₂N-CHPh-CHPh-OH, H₂N-CHMe-CHPh-OH, MeHN-CHMe-CHPh-OH oder TsNH-CHPh-CHPh-NH₂, insbesondere durch einfache Deprotonierung von (1S,2S)-N-p-Toluolsulfonyl-1,2-diphenylethylendiamin oder (1R,2R)-N-p-Toluolsulfonyl-1,2-diphenylethylendiamin erhältlich ist.

Deshalb sieht eine Ausgestaltung des Verfahrens nach der Erfindung vor, dass der ausgewählte Ligand, insbesondere der aus Aminen ausgewählte Ligand deprotoniert wird, vorzugsweise einmal deprotoniert wird.

In einer abgewandelten Ausführung sieht das erfinderische Verfahren vor, dass der Übergangsmetallkatalysator wenigstens einen Liganden enthält, der ausgewählt ist aus der Gruppe bestehend aus H₂N-CH₂-CH₂-OH, MeHN-CH₂-CH₂-OH, H₂N-CH₂-CH₂-NH₂, TsNH-CH₂-CH₂-NH₂, TsNH-CH₂-CH₂-NH-(CH₂)ₙ-Oₘ-(CH₂)ₒ-Aryl mit n = 1 - 4, m = 0 oder 1 und o = 1 - 4 und Aryl = Phenyl oder Mono-, Di-, Tri-C1 - C4-Alkylphenyl, optisch aktiver chiraler Baustein, wobei dieser Ligand optional über einen Linker mit einem Aromaten verbunden ist.

Eine noch andere Ausgestaltung des erfinderischen Verfahrens bestimmt, dass der Übergangsmetallkatalysator wenigstens einen Liganden enthält, der ausgewählt ist aus der Gruppe bestehend aus einem optisch aktiven Amin, insbesondere H₂N-CHPh-CHPh-OH, H₂N-CHMe-CHPh-OH, MeHN-CHMe-CHPh-OH, TsNH-CHPh-CHPh-NH₂, (1S,2S)-N-p-Touluolsulfonyl-1,2-diphenylethylendiamin, (1R,2R)- N-p-Touluolsulfonyl-1,2-diphenylethylendiamin, N-[(1S,2S)-1,2-Diphenyl-2-(2-(4-methylbenzyloxy)ethylamino)-ethyl]-4-methylbenzol sulfonamid, N-[(1R,2R)-1,2-Diphenyl-2-(2-(4-methylbenzyloxy)ethylamino)-ethyl]-4-methylbenzol sulfonamid, einer optisch aktiven Aminosäure; und der bevorzugt ausgewählt ist aus der Gruppe bestehend aus H₂N-CHPh-CHPh-OH, H₂N-CHMe-CHPh-OH, MeHN-CHMe-CHPh-OH, TsNH-CHPh-CHPh-NH₂, (1S,2S)-N-p-Touluolsulfonyl-1,2- diphenylethylendiamin, (1R,2R)- N-p-Touluolsulfonyl-1,2-diphenylethylendiamin, N-[(1S,2S)-1,2-Diphenyl-2-(2-(4-methylbenzyloxy)ethylamino)-ethyl]-4-methylbenzol sulfonamid, N-[(1R,2R)-1,2-Diphenyl-2-(2-(4-methylbenzyloxy)ethylamino)-ethyl]-4-methylbenzol sulfonamid, wobei dieser Ligand optional über einen Linker mit einem Aromaten verbunden ist.

Übergangsmetallkatalysatoren von einem Reaktionsgemisch abzutrennen, bedeutet einen zusätzlichen Filtrations- oder Extraktionsschritt. Dieser Schritt ist bei vielen dieser Übergangsmetallkatalysatoren unabdingbar, weil sie bei jedweder Fixierung oder Immobilisierung ihre katalytische Aktivität teilweise oder vollständig verlieren würden. Einigen Übergangsmetallkatalysatoren macht eine solche Fixierung jedoch nichts aus.

Deshalb sieht eine sehr ökonomisch ausgelegte Variante des erfinderischen Verfahrens vor, dass das Übergangsmetall auf einem festen Träger aufgebracht ist; bevorzugt auf einem festen Träger, der wenigstens einen Stoff enthält, der ausgewählt ist aus der Gruppe bestehend aus Kohlenstoff, Aluminiumoxid und Siliziumdioxid; und am bevorzugtesten auf einem festen Träger, der aus wenigstens einem Stoff aufgebaut ist, der ausgewählt ist aus der Gruppe bestehend aus Kohlenstoff, Aluminiumoxid und Siliziumdioxid.

Mit dieser Ausführung wird eine Abtrennung des Übergangsmetallkatalysators vom Reaktionsprodukt Astaxanthin 1 im Sinn eines separaten Verfahrensschritts vermieden.

Die Reduktion von Astacin 2 wurde bei sehr unterschiedlichen pH-Werten versucht mit dem Ergebnis, dass eine vollständige oder nahezu vollständige Umsetzung zum Astaxanthin 1 nur in einem basischen Milieu möglich ist.

Ein weiterer Aspekt der Erfindung sieht deshalb vor, dass Astacin 2 im Basischen, bevorzugt in einem pH-Bereich von 8 bis 12, mit einem Reduktionsmittel stereounselektiv oder stereoselektiv umgesetzt wird.

Als Basen dienen entweder die bereits oben angegebenen Amin Liganden und/oder es wird zusätzlich Base zugegeben.

Als Base, insbesondere als zusätzliche Base, verwendet man Ammoniak, Trimethylamin, Triethylamin, Tri-n-propylamin, Tri-n-butylamin, Diisopropylethylamin oder eine Mischung von wenigstens zweier dieser Verbindungen.

Deshalb bestimmt eine weiter präzisierte Ausführung der Erfindung, dass Astacin 2 im Basischen, bevorzugt in einem pH-Bereich von 8 bis 12, mit einem Reduktionsmittel stereounselektiv oder stereoselektiv umgesetzt wird, wobei hierzu Basen eingesetzt werden die aus der Gruppe bestehend aus Ammoniak, Trimethylamin, Triethylamin, Tri-n-propylamin, Tri-n-butylamin, Diisopropylethylamin oder einer Mischung von wenigstens zweier dieser Verbindungen ausgewählt sind.

Im Besonderen bedeutet dies, ein Verfahren zur Herstellung von Astaxanthin der Formel 1 bereitzustellen 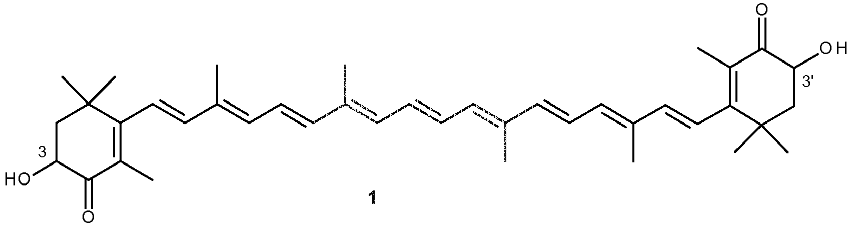 wobei das Asymmetriezentrum in Position 3 und 3' racemisch, oder (S)- oder (R)- konfiguriert ist und die exozyklischen Dopppelbindungen entweder E oder E und/oder Z konfiguriert sind, bei dem erfindungsgemäß Astacin der Formel 2, 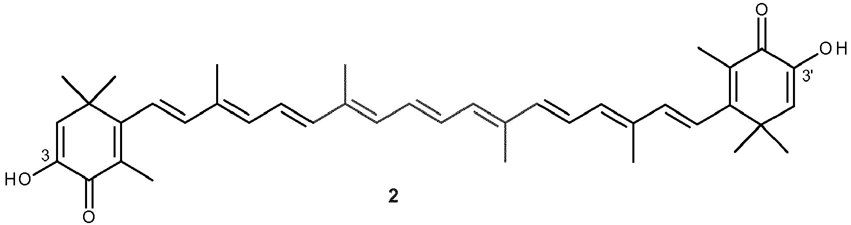 bei dem die exozyklischen Dopppelbindungen entweder E oder E und/oder Z konfiguriert sind, im Basischen, bevorzugt in einem pH-Bereich von 8 bis 12, mit einem Reduktionsmittel, das ausgewählt ist aus der Gruppe bestehend aus Ameisensäure und/oder den Salzen der Ameisensäure, Isopropanol, Butan-2-ol, in Gegenwart eines Übergangsmetallkatalysators stereounselektiv oder stereoselektiv umgesetzt wird, wobei der Übergangsmetallkatalysator als Übergangsmetall Ruthenium (Ru) enthält und wenigstens einen Liganden, der ausgewählt ist aus der Gruppe bestehend aus H₂N-CH₂-CH₂-OH, MeHN-CH₂-CH₂-OH, H₂N-CH₂-CH₂-NH₂, TsNH-CH₂-CH₂-NH₂, TsNH-CH₂-CH₂-NH-(CH₂)ₙ-Oₘ-(CH₂)ₒ-Aryl mit n = 1 - 4, m = 0 oder 1 und o = 1 - 4 und Aryl = Phenyl oder Mono-, Di-, Tri-C1 - C4-Alkylphenyl, optisch aktive Verbindung.

Eine Variante zum Hervorbringen enantiomeren-/ respektive diastereomerenangereicherter oder enantiomeren-/ respektive diastereomerenreiner Verbindungen sieht ein Verfahren zur Herstellung von Astaxanthin der Formel 1 vor, 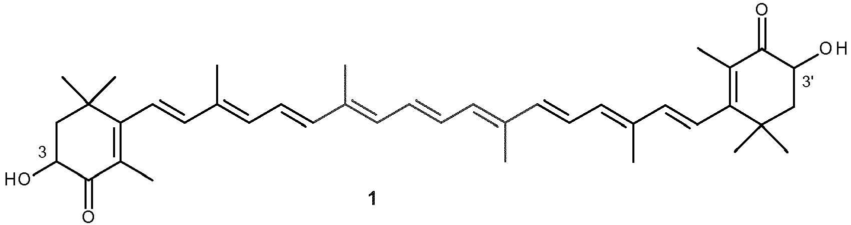 wobei das Asymmetriezentrum in Position 3 und 3' (S)- oder (R)- konfiguriert ist und die exozyklischen Dopppelbindungen entweder E oder E und/oder Z konfiguriert sind, bei dem man erfindungsgemäß Astacin der Formel 2 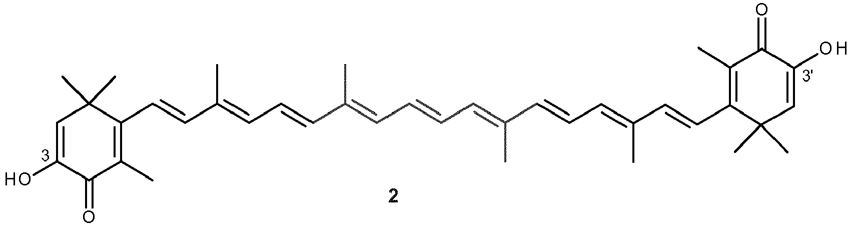 bei dem die exozyklischen Dopppelbindungen entweder E oder E und/oder Z konfiguriert sind, im Basischen, bevorzugt in einem pH-Bereich von 8 bis 12, mit einem Reduktionsmittel, das ausgewählt ist aus der Gruppe bestehend aus Ameisensäure, den Salzen der Ameisensäure, Isopropanol oder Butan-2-ol, in Gegenwart eines optisch aktiven Übergangsmetallkatalysators stereoselektiv umsetzt, wobei der optisch aktive Übergangsmetallkatalysator als Übergangsmetall Ruthenium (Ru) enthält und wenigstens einen Liganden, der ausgewählt ist aus der Gruppe bestehend aus H₂N-CHPh-CHPh-OH, H₂N-CHMe-CHPh-OH, MeHN-CHMe-CHPh-OH, TsNH-CHPh-CHPh-NH₂, (1S,2S)-N-p-Touluolsulfonyl-1,2-diphenylethylendiamin, (1R,2R)- N-p-Touluolsulfonyl-1,2-diphenylethylendiamin, N-[(1S,2S)-1,2-Diphenyl-2-(2-(4-methylbenzyl-oxy)ethylamino)-ethyl]-4-methylbenzol sulfonamid, N-[(1R,2R)-1,2-Diphenyl-2-(2-(4-methylbenzyloxy)ethylamino)-ethyl]-4-methylbenzol sulfonamid, einer optisch aktiven Aminosäure.

Sollte ein erfinderischer Übergangsmetallkatalysator nicht in der Lage sein, in geträgerter Form seine Aktivität beizubehalten, muss in flüssiger Phase gearbeitet werden.

Das erfindungsgemäße Verfahren wird deshalb in vielen Fällen üblicherweise in flüssiger Phase, das heißt in mindestens einem Lösemittel oder Lösemittelgemisch durchgeführt. Bevorzugt enthält die flüssige Phase mindestens ein organisches Lösemittel, wobei die flüssige Phase üblicherweise zu mehr als 50 Volumen-% aus organischen Lösemitteln besteht

Eine weiter ausgestaltete Variante der Erfindung sieht deshalb vor, dass das Astacin 2 in einem flüssigen Medium mit einem Reduktionsmittel stereounselektiv oder stereoselektiv umgesetzt wird, bevorzugt in einem flüssigen Medium, das zu mehr als 50 Volumen-% aus wenigstens einem organischen Lösemittel besteht.

Unter einem flüssigen Medium versteht man jede ein- oder mehrphasige flüssige Zusammensetzung aus einem Lösemittel oder einem Lösemittelgemisch. Das flüssige Medium ist deshalb ausgewählt aus der Gruppe Dichlormethan, Ethylenglycoldimethylether, Diethylenglycoldimethylether, Tetrahydrofuran, Ethylencarbonat, Propylencarbonat, Dimethylformamid, Dimethylsulfoxid, Ethylacetat, n-Propylacetat, Toluol, Xylol, Heptan, Hexan, Pentan, N-Methyl-2-pyrrolidon, Dioxan, 2-Methyl-Tetrahydrofuran, Acetonitril, Methyl-tert.-butylether, Diisopropylether, Diethylether, Di-n-butylether, Wasser oder aus einer Mischung wenigstens zweier dieser Lösemittel.

Wenn der Anteil an organischem Lösemittel größer als 50 Volumen-% gewählt wird, lösen sich Reaktionsedukte und Reaktionsprodukte so gut, das eine schnelle Reaktion gewährleistet ist.

Bei der Umsetzung des Astacins 2 mit dem Reduktionsmittel in Gegenwart des Übergangsmetallkatalysators fallen oft Salze an, gerade bei einer Umsetzung im Basischen. Diese lösen sich meist gut in Wasser und lassen sich dann leicht abtrennen. Als anorganisches Lösemittel kann das flüssige Medium deshalb insbesondere Wasser enthalten.

Eine noch andere Variante des erfinderischen Verfahrens bestimmt mithin, dass das Astacin 2 in einem flüssigen Medium mit einem Reduktionsmittel stereounselektiv oder stereoselektiv umgesetzt wird, bevorzugt in einem flüssigen Medium, das zu mehr als 50 Volumen-% aus wenigstens einem organischen Lösemittel besteht und als anorganisches Lösemittel Wasser enthält.

Je nach Zusammensetzung des flüssigen Mediums aus unterschiedlichen Lösemitteln kann das flüssige Medium ein 1-Phasen-, 2-Phasen- oder auch Mehrphasensystem darstellen.

Löslichkeiten und mithin Reaktionsgeschwindigkeiten unterscheiden sich von Ausgangsverbindung zu Ausgangsverbindung. Bei vielfältigen Versuchen haben sich jedoch einige Lösemittel oder Mischungen von ihnen als besonders geeignet herausgestellt. Als Lösemittel werden insbesondere Gemische aus Dichlormethan, Ethylenglycoldimethylether, Diethylenglycoldimethylether, sowie THF und Wasser eingesetzt.

Eine Weiterleitung der Erfindung stellt deshalb unter Schutz, dass das organische Lösemittel wenigstens eine Verbindung enthält, die ausgewählt ist aus der Gruppe bestehend aus Dichlormethan, Ethylenglycoldimethylether, Diethylenglycoldimethylether, Tetrahydrofuran, Ethylencarbonat, Propylencarbonat, Dimethylformamid, Dimethylsulfoxid, Ethylacetat, n-Propylacetat, Toluol, Xylol, Heptan, Hexan, Pentan, N-Methyl-2-pyrrolidon, Dioxan, 2-Methyltetrahydrofuran, Methyl-tert.-butylether, Diisopropylether, Diethylether, Di-n-butylether, Acetonitril und bevorzugt aus der Gruppe bestehend aus Dichlormethan, Ethylenglycoldimethylether, Diethylenglycoldimethylether, Tetrahydrofuran, Acetonitril, Ethylencarbonat, Propylencarbonat.

Bei vielfältigen Tests hat sich nachfolgende Ausführung als besonders geeignet in Bezug auf Ausbeute und Reinheit des erhaltenen Astaxanthins 1 erwiesen. Sie bestimmt, dass das Astacin 2 in einem flüssigen Medium mit einem Reduktionsmittel stereounselektiv oder stereoselektiv umgesetzt wird, bevorzugt in einem flüssigen Medium, das zu mehr als 50 Volumen-% aus wenigstens einem organischen Lösemittel ausgewählt aus der Gruppe bestehend aus Dichlormethan, Ethylenglycoldimethylether, Diethylenglycoldimethylether, Tetrahydrofuran, Acetonitril, Propylencarbonat, Ethylencarbonat besteht und als anorganisches Lösemittel Wasser enthält.

Eine weitere Fortführung der Erfindung führt nach kurzer Reaktionszeit zu hohen Ausbeuten. Sie umfasst ein Verfahren zur Herstellung von Astaxanthin der Formel 1 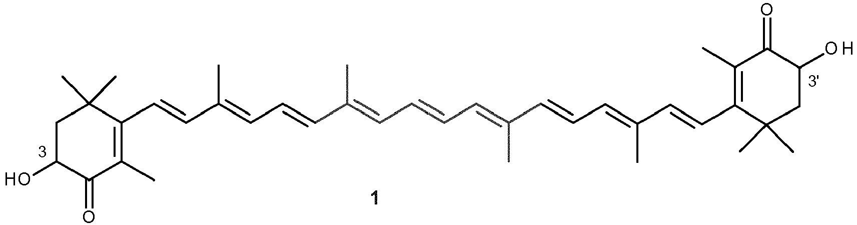 wobei das Asymmetriezentrum in Position 3 und 3' racemisch, oder (S)- oder (R)- konfiguriert ist und die exozyklischen Dopppelbindungen entweder E oder E und/oder Z konfiguriert sind, bei dem erfindungsgemäß Astacin der Formel 2 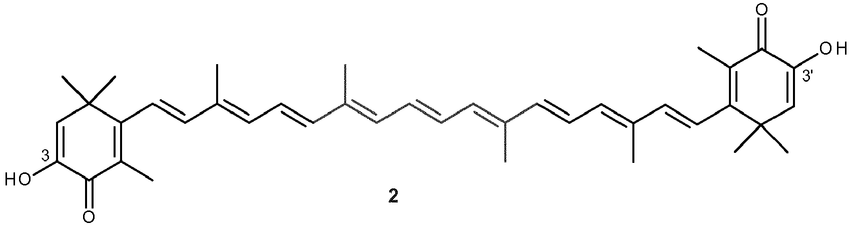 bei dem die exozyklischen Dopppelbindungen entweder E oder E und/oder Z konfiguriert sind, im Basischen, bevorzugt in einem pH-Bereich von 8 bis 12, in einem flüssigen Medium, mit einem Reduktionsmittel, das ausgewählt ist aus der Gruppe bestehend aus Ameisensäure und/oder den Salzen der Ameisensäure, Isopropanol, Butan-2-ol, in Gegenwart eines Übergangsmetallkatalysators stereounselektiv oder stereoselektiv umgesetzt wird, wobei der Übergangsmetallkatalysator als Übergangsmetall Ruthenium (Ru) enthält und wenigstens einen Liganden, der ausgewählt ist aus der Gruppe bestehend aus H₂N-CH₂-CH₂-OH, MeHN-CH₂-CH₂-OH, H₂N-CH₂-CH₂-NH₂, TsNH-CH₂-CH₂-NH₂, TsNH-CH₂-CH₂-NH-(CH₂)ₙ-Oₘ-(CH₂)ₒ-Aryl mit n = 1 - 4, m = 0 oder 1 und o = 1 - 4 und Aryl = Phenyl oder Mono-, Di-, Tri-C1 - C4-Alkylphenyl, optisch aktive Verbindung und wobei das flüssige Medium zu mehr als 50 Volumen % aus wenigstens einem organischen Lösemittel besteht, das ausgewählt ist aus der Gruppe bestehend aus Dichlormethan, Ethylenglycoldimethylether, Diethylenglycoldimethylether, Tetrahydrofuran, Acetonitril, Propylencarbonat, Ethylencarbonat.

Dies gilt auch für die Variante zur Erzeugung hoch enantiomeren-/ respektive diastereomerenangereicherter oder entantiomeren-/ respektive diastereomerenreiner Verbindungen. Sie beschreibt ein Verfahren zur Herstellung von Astaxanthin der Formel 1, 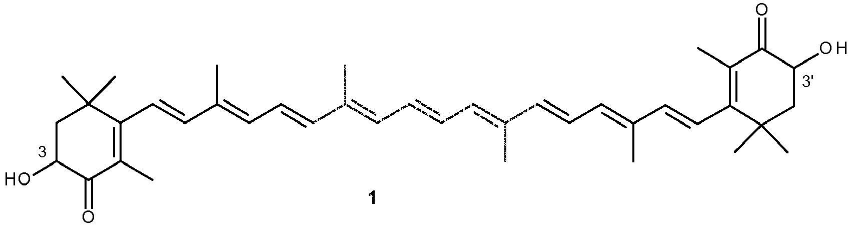 wobei das Asymmetriezentrum in Position 3 und 3' (S)- oder (R)- konfiguriert ist und die exozyklischen Dopppelbindungen entweder E oder E und/oder Z konfiguriert sind, bei dem man erfindungsgemäß Astacin der Formel 2 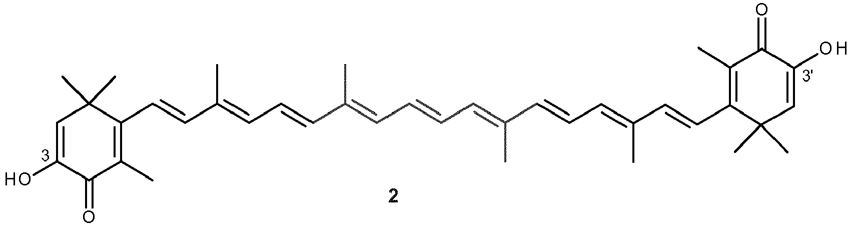 bei dem die exozyklischen Dopppelbindungen entweder E oder E und/oder Z konfiguriert sind, im Basischen, bevorzugt in einem pH-Bereich von 8 bis 12, in einem flüssigen Medium mit einem Reduktionsmittel, das ausgewählt ist aus der Gruppe bestehend aus Ameisensäure, den Salzen der Ameisensäure, Isopropanol oder Butan-2-ol, in Gegenwart eines optisch aktiven Übergangsmetallkatalysators stereoselektiv umsetzt, wobei der optisch aktive Übergangsmetallkatalysator als Übergangsmetall Ruthenium (Ru) enthält und wenigstens einen Liganden, der ausgewählt ist aus der Gruppe bestehend aus H₂N-CHPh-CHPh-OH, H₂N-CHMe-CHPh-OH, MeHN-CHMe-CHPh-OH, TsNH-CHPh-CHPh-NH₂, (1S,2S)-N-p-Touluolsulfonyl-1,2-diphenylethylendiamin, (1R,2R)- N-p-Touluolsulfonyl-1,2-diphenylethylendiamin, N-[(1S,2S)-1,2-Diphenyl-2-(2-(4-methylbenzyloxy)ethylamino)-ethyl]-4-methylbenzol sulfonamid, N-[(1R,2R)-1,2-Diphenyl-2-(2-(4-methylbenzyloxy)ethylamino)-ethyl]-4-methylbenzol sulfonamid, einer optisch aktiven Aminosäure und wobei das flüssige Medium zu mehr als 50 Volumen-% aus wenigstens einem organischen Lösemittel besteht, das ausgewählt ist aus der Gruppe bestehend aus Dichlormethan, Ethylenglycoldimethylether, Diethylenglycoldimethylether, Tetrahydrofuran, Acetonitril, Propylencarbonat, Ethylencarbonat.

Ein weiterer Vorteil des erfinderischen Verfahrens ist, dass es auch bei niedrigen Temperaturen in angemessener Zeit hohe Ausbeuten an Astaxanthin 1 hervorbringt. Deshalb ist Gegenstand einer weiteren erfinderischen Abwandlung, dass man das Astacin 2 bei einer Temperatur von 10 °C bis 85 °C; bevorzugt von 20 °C bis 60 °C, mit einem Reduktionsmittel stereounselektiv oder stereoselektiv umsetzt.

Selbstverständlich kann das erfinderische Verfahren auch unter Druck durchgeführt werden. Der bevorzugte Druckbereich liegt zwischen 0 und 10 bar. Auch unter Druck liegt der besonders bevorzugte Temperaturbereich bei 20 bis 60 °C.

Die Reaktion kann diskontinuierlich in der batch- oder semi-batch-Fahrweise oder kontinuierlich in den für den Fachmann üblichen Apparaten durchgeführt werden. Als Beispiel seien genannt, Rührkessel, Rührkesselkaskade und Rohrreaktor.

Die Aufarbeitung erfolgt ebenfalls unter den üblichen Methoden. Bevorzugt ist die der Extraktion und Kristallisation.

Astacin 2 lässt sich für die Synthese von Astaxanthin 1 einsetzen, wobei unter den Begriff Astaxanthin sowohl racemische Gemische, als auch die Mesoform und alle enantiomerenreinen Vertreter des Astaxanthins fallen.

Deshalb ist auch die Verwendung von Astacin 2 als Intermediat zur Herstellung von (3R/S, 3'R/S)-Astaxanthin 1a, wobei die exozyklischen Dopppelbindungen in 1a entweder E oder E und/oder Z konfiguriert sind 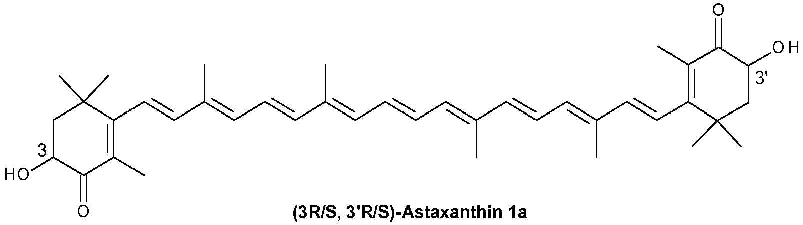 und/oder von (3S, 3'S)-Astaxanthin 1b, wobei die exozyklischen Dopppelbindungen in 1b entweder E oder E und/oder Z konfiguriert sind 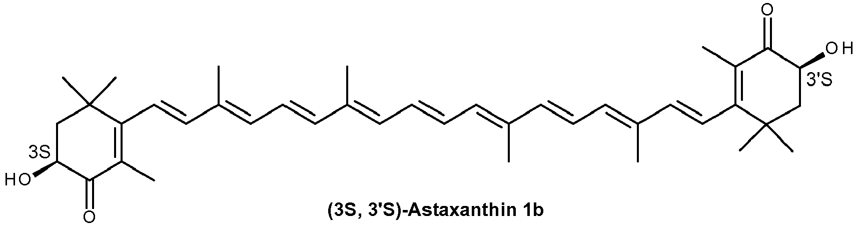 und/oder von (3R, 3'R)-Astaxanthin 1c, wobei die exozyklischen Dopppelbindungen in 1c entweder E oder E und/oder Z konfiguriert sind

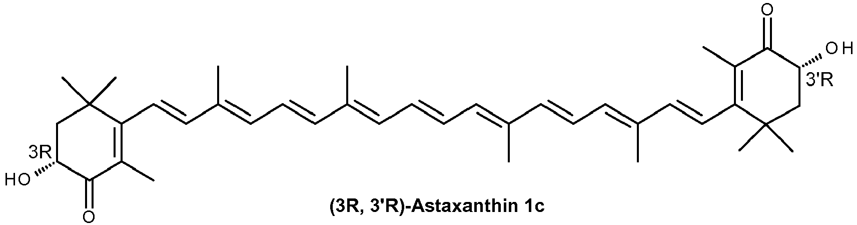

### Gegenstand der Erfindung.

Weitere Merkmale, Einzelheiten und Vorteile der Erfindung ergeben sich aus dem Wortlaut der Ansprüche sowie aus der folgenden Beschreibung von Ausführungsbeispielen.

### Beispiele:

### Beispiel 1: Synthese von (3S,3'S)-Astaxanthin (3S,3'S)-1 aus Astacin 2

0,5 g (0,8 mmol) Astacin 2 werden in 20 ml Dichlormethan bei 22 °C vorgelegt, 1,01 g (10,02 mmol) Triethylamin und 5,1 mg (0,01 mmol) Chlor{[(1S,2S)-(+)-2-amino-1,2-diphenylethyl](4-toluolsulfonyl)amido}(p-cymol)ruthenium(II) zugegeben, 0,37 g (8,01 mmol) Ameisensäure zugetropft und der Ansatz für 7,5 h auf 40 °C erwärmt. Nach dem Abkühlen auf 20 °C gibt man 10 ml Wasser zu, trennt die Phasen, und wäscht die organische Phase mit 10 ml Wasser. Anschließend wird diese am Rotationsverdampfer eingeengt. Der Rückstand wird suspendiert in 2,7 ml Methanol für 4 h auf 106 °C erhitzt und anschließend wieder eingeengt. Es werden 0,523 g (71,38 %ig, Ausbeute: 78 %) (3S,3'S)-Astaxanthin 1b mit einem Diastereomerenüberschuss von 98 % und einem Enantiomerenüberschuss von >99 % erhalten.

### Beispiel 2 Synthese von (3R/S,3'R/S)-Astaxanthin (3R/S,3'R/S)-1 aus Astacin 2

1,0 g (1,67 mmol) Astacin 2 werden in 20 ml Dichlormethan bei 22 °C vorgelegt, 2,11 g (20,85 mmol) Triethylamin und 8,09 mg (0,02 mmol) Chlor{[2-amino-ethyl](4-toluolsulfonyl)amido}(p-cymol)ruthenium(II) zugegeben, 0,77 g (16.68 mmol) Ameisensäure zugetropft und der Ansatz für 4,5 h auf 40 °C erwärmt. Nach dem Abkühlen auf 20 °C gibt man 15 ml Wasser zu, trennt die Phasen, und wäscht die organische Phase mit 15 ml Wasser. Anschließend wird diese am Rotationsverdampfer eingeengt. Der Rückstand wird suspendiert in 6 ml Methanol für 4 h auf 106 °C erhitzt und anschließend wieder eingeengt. Es werden 0,72 g (88,1 %ig, Ausbeute: 63,7 %) (3R/S,3'R/S)-Astaxanthin 1a erhalten.

### Beispiel 3:

2,0 g (3,31 mmol) Astacin 2 werden in 40 ml Dichlormethan bei 22 °C vorgelegt, 4,19 g (41,4 mmol) Triethylamin und 21,1 mg (0,03 mmol) Chlor{[(1R,2R)-(+)-2-amino-1,2-diphenylethyl](4-toluolsulfonyl)amido}(p-cymol)ruthenium(II) zugegeben und 1,51 g (33,1 mmol) Ameisensäure bei 22 - 35 °C zugetropft. Das Reaktionsgemisch wird 7 h auf 40 °C erwärmt. Der Ansatz wird mit 15 ml Wasser versetzt und die Phasen getrennt. Die organische Phase wird mit 40 ml 10 %ige Essigsäure und 40 ml ges. Natriumhydrogencarbonat-Lösung gewaschen und dann am Rotationsverdampfer eingeengt. Der Rückstand wird in 12 ml Methanol suspendiert für 4 h auf 106 °C erhitzt und nach dem Abkühlen auf 0 °C abfiltriert, mit 3 ml Methanol gewaschen und in Vakuumtrockenschrank bei 20 °C getrocknet. Es werden 1,21 g (93,3 %ig, Ausbeute: 57 %) (3R,3'R)-Astaxanthin 1c mit einem Diastereomerenüberschuss von 97 % und einem Enantiomerenüberschuss von >99 % erhalten.

Man erkennt, dass die Erfindung ein Verfahren zur stereounselektiven sowie zur stereoselektiven Synthese von Astaxanthin aus Astacin betrifft. Hierzu setzt man ein Reduktionsmittel ein, das ausgewählt ist aus der Gruppe Wasserstoffgas, einem sekundären Alkohol, Ameisensäure sowie den Salzen der Ameisensäure oder aus einem Gemisch wenigstens zweier Vertreter der vorab angegebenen Verbindungsklassen. Ein weiterer Gegenstand der Erfindung ist die Verwendung von Astacin als Ausgangsverbindung für die Synthese von Astaxanthin.

## Patentansprüche

1. Verfahren zur Herstellung von Astaxanthin der Formel (1) 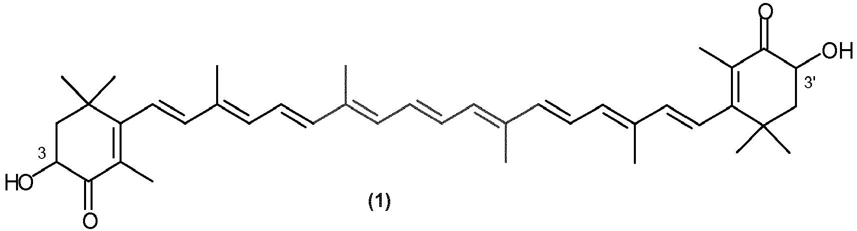 wobei das Asymmetriezentrum in Position 3 und 3' racemisch oder jeweils (S)- oder (R)- konfiguriert ist und die exozyklischen Dopppelbindungen entweder E oder E und/oder Z konfiguriert sind, **dadurch gekennzeichnet, dass** Astacin der Formel (2), 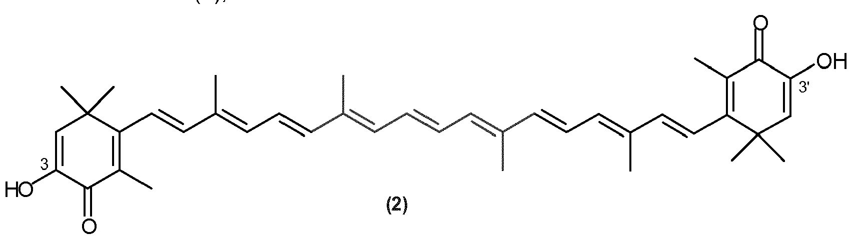 bei dem die exozyklischen Dopppelbindungen entweder E oder E und/oder Z konfiguriert sind, mit einem Reduktionsmittel stereounselektiv oder stereoselektiv umgesetzt wird,
wobei das Reduktionsmittel wenigstens eine Verbindung ist, die ausgewählt ist aus der Gruppe bestehend aus einem sekundären Alkohol; Ameisensäure, den Salzen der Ameisensäure.

2. Verfahren nach Anspruch 1, **dadurch gekennzeichnet, dass** das Astacin (2) mit dem Reduktionsmittel in Gegenwart eines Übergangsmetallkatalysators, stereounselektiv oder stereoselektiv umgesetzt wird.

3. Verfahren nach Anspruch 2, **dadurch gekennzeichnet, dass** der Übergangsmetallkatalysator ein Übergangsmetall enthält, das ausgewählt ist aus der Gruppe bestehend aus Ti, Zr, Hf, V, Nb, Ta, Cr, Mo, W, Mn, Re, Fe, Ru, Os, Co, Rh, Ir, Ni, Pd, Pt, Cu, Ag und Au.

4. Verfahren nach Anspruch 2 oder 3, **dadurch gekennzeichnet, dass** der Übergangsmetallkatalysator wenigstens einen Liganden enthält, der ausgewählt ist aus Aminen und/oder Phosphanen.

5. Verfahren nach Anspruch 4, **dadurch gekennzeichnet, dass** der Ligand ein Phosphan der allgemeinen Formel (3) ist, 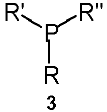 wobei R, R' und R" unabhängig voneinander ausgewählt sind aus der Gruppe bestehend aus wenigstens einem der Reste C1-C4-Alkyl, Phenyl, ein bis dreifach C1 - C4-Alkyl-substituiertes Aryl.

6. Verfahren nach einem der Ansprüche 2 bis 5, **dadurch gekennzeichnet, dass** der Übergangsmetallkatalysator wenigstens einen Liganden enthält, der ausgewählt ist aus der Gruppe bestehend aus H₂N-CH₂-CH₂-OH, MeHN-CH₂-CH₂-OH, H₂N-CH₂-CH₂-NH₂, TsNH-CH₂-CH₂-NH₂,TsNH-CH₂-CH₂-NH-(CH₂)ₙ-Oₘ-(CH₂)ₒ-Aryl mit n = 1 - 4, m = 0 oder 1 und o = 1 - 4 und Aryl = Phenyl oder Mono-, Di-, Tri-C1 - C4-Alkylphenyl, optisch aktiver Verbindung.

7. Verfahren nach einem der Ansprüche 2 bis 5, **dadurch gekennzeichnet, dass** der Übergangsmetallkatalysator wenigstens einen Liganden enthält, der ausgewählt ist aus der Gruppe bestehend aus einem optisch aktiven Amin, insbesondere H₂N-CHPh-CHPh-OH, H₂N-CHMe-CHPh-OH, MeHN-CHMe-CHPh-OH, TsNH-CHPh-CHPh-NH₂, (1S,2S)-N-p-Touluolsulfonyl-1,2-diphenylethylendiamin, (1R,2R)- N-p-Touluolsulfonyl-1,2-diphenylethylendiamin, N-[(1S,2S)-1,2-Diphenyl-2-(2-(4-methylbenzyloxy)ethylamino)-ethyl]-4-methylbenzol sulfonamid, N-[(1R,2R)-1,2-Diphenyl-2-(2-(4-methylbenzyloxy)ethylamino)-ethyl]-4-methylbenzol sulfonamid, einer optisch aktiven Aminosäure.

8. Verfahren nach Anspruch 4, 6 oder 7, **dadurch gekennzeichnet, dass** der Ligand deprotoniert wird.

9. Verfahren nach einem der Ansprüche 3 bis 8, **dadurch gekennzeichnet, dass** das Übergangsmetall auf einem festen Träger aufgebracht ist.

10. Verfahren nach einem der Ansprüche 1 bis 9, **dadurch gekennzeichnet, dass** das Astacin (2) im Basischen mit einem Reduktionsmittel stereounselektiv oder stereoselektiv umgesetzt wird.

11. Verfahren nach einem der Ansprüche 1 bis 10, **dadurch gekennzeichnet, dass** das Astacin (2) in einem flüssigen Medium mit einem Reduktionsmittel stereounselektiv oder stereoselektiv umgesetzt wird, bevorzugt in einem flüssigen Medium, das zu mehr als 50 Volumen-% aus wenigstens einem organischen Lösemittel besteht.

12. Verfahren nach Anspruch 11, **dadurch gekennzeichnet, dass** das organische Lösemittel wenigstens eine Verbindung enthält, die ausgewählt ist aus der Gruppe bestehend aus Dichlormethan, Ethylenglycoldimethylether, Diethylenglycoldimethylether, Tetrahydrofuran, Acetonitril, Ethylencarbonat und Propylencarbonat.

13. Verfahren nach einem der Ansprüche 1 bis 12, **dadurch gekennzeichnet, dass** das Astacin (2) bei einer Temperatur von 10 °C bis 85 °C mit einem Reduktionsmittel stereounselektiv oder stereoselektiv umgesetzt wird.

14. Verwendung von Astacin (2) als Intermediat zur Herstellung von (3R/S, 3'R/S)-Astaxanthin (1a), wobei die exozyklischen Dopppelbindungen in (1a) entweder E oder E und/oder Z konfiguriert sind 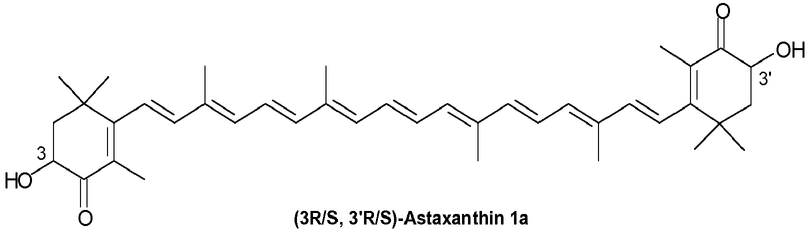 und/oder von (3S, 3'S)-Astaxanthin (1b), wobei die exozyklischen Dopppelbindungen in (1b) entweder E oder E und/oder Z konfiguriert sind 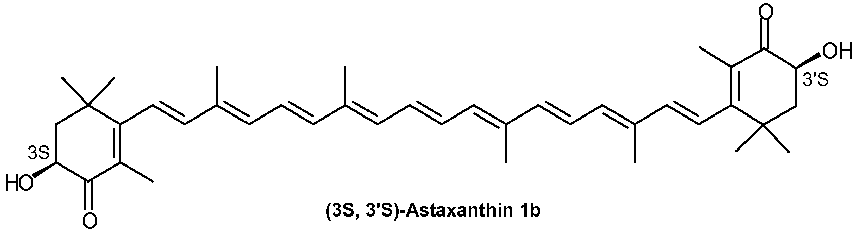 und/oder von (3R, 3'R)-Astaxanthin (1c), wobei die exozyklischen Dopppelbindungen in (1c) entweder E oder E und/oder Z konfiguriert sind (1c)

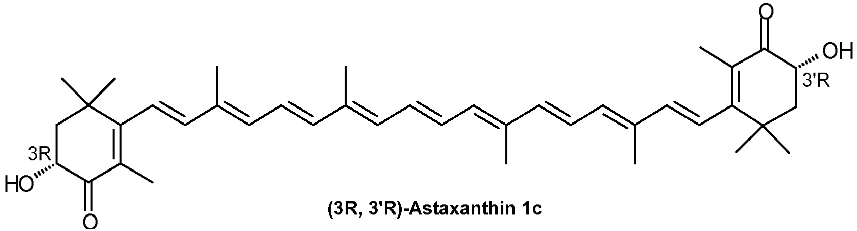

## Claims

1. A method for preparing astaxanthin of the formula (1) 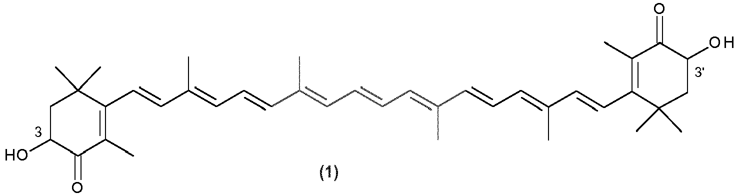 in which the asymmetric center in position 3 and 3' is racemic or each has (S) or (R) configuration and the exocyclic double bonds have either E or E and/or Z configuration, **wherein** astacin of the formula (2), 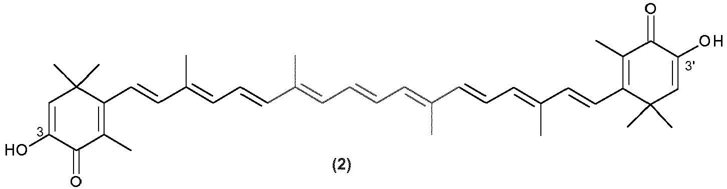 in which the exocyclic double bonds have either E or E and/or Z configuration, is reacted non-stereoselectively or stereoselectively with a reducing agent,
wherein the reducing agent is at least one compound selected from the group consisting of a secondary alcohol; formic acid, the salts of formic acid.

2. The method according to claim 1, **wherein** the astacin (2) is reacted non-stereoselectively or stereoselectively with the reducing agent in the presence of a transition metal catalyst.

3. The method according to claim 2, **wherein** the transition metal catalyst comprises a transition metal selected from the group consisting of Ti, Zr, Hf, V, Nb, Ta, Cr, Mo, W, Mn, Re, Fe, Ru, Os, Co, Rh, Ir, Ni, Pd, Pt, Cu, Ag and Au.

4. The method according to claim 2 or 3, **wherein** the transition metal catalyst comprises at least one ligand selected from amines and/or phosphanes.

5. The method according to claim 4, **wherein** the ligand is a phosphane of the general formula (3), 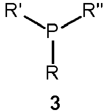 where R, R' and R" are each independently selected from the group consisting of at least one of the residues C1-C4-alkyl, phenyl, mono- up to tri-C1 - C4-alkyl-substituted aryl.

6. The method according to any of claims 2 to 5, **wherein** the transition metal catalyst comprises at least one ligand selected from the group consisting of H₂N-CH₂-CH₂-OH, MeHN-CH₂-CH₂-OH, H₂N-CH₂-CH₂-NH₂, TsNH-CH₂-CH₂-NH₂, TsNH-CH₂-CH₂-NH-(CH₂)ₙ-Oₘ-(CH₂)ₒ-aryl where n = 1 - 4, m = 0 or 1 and o = 1 - 4 and aryl = phenyl or mono-, di-, tri-C1 - C4-alkylphenyl, optically active compound.

7. The method according to any of claims 2 to 5, **wherein** the transition metal catalyst comprises at least one ligand selected from the group consisting of an optically active amine, in particular H₂N-CHPh-CHPh-OH, H₂N-CHMe-CHPh-OH, MeHN-CHMe-CHPh-OH, TsNH-CHPh-CHPh-NH₂, (1S,2S)-N-p-toluenesulfonyl-1,2-diphenylethylenediamine, (1R,2R)- N-p-toluenesulfonyl-1,2-diphenylethylenediamine, N-[(1S,2S)-1,2-diphenyl-2-(2-(4-methylbenzyloxy)ethylamino)ethyl]-4-methylbenzene sulfonamide, N-[(1R,2R)-1,2-diphenyl-2-(2-(4-methylbenzyloxy)ethylamino)ethyl]-4-methylbenzene sulfonamide, an optically active amino acid.

8. The method according to claim 4, 6 or 7, **wherein** the ligand is deprotonated.

9. The method according to any of claims 3 to 8, **wherein** the transition metal is applied to a solid support.

10. The method according to any of claims 1 to 9, **wherein** the astacin (2) is reacted non-stereoselectively or stereoselectively with a reducing agent under basic conditions.

11. The method according to any of claims 1 to 10, **wherein** the astacin (2) is reacted non-stereoselectively or stereoselectively with a reducing agent in a liquid medium, preferably in a liquid medium comprising more than 50% by volume of at least one organic solvent.

12. The method according to claim 11, **wherein** the organic solvent comprises at least one compound selected from the group consisting of dichloromethane, ethylene glycol dimethyl ether, diethylene glycol dimethyl ether, tetrahydrofuran, acetonitrile, ethylene carbonate and propylene carbonate.

13. The method according to any of claims 1 to 12, **wherein** the astacin (2) is reacted non-stereoselectively or stereoselectively with a reducing agent at a temperature of 10°C to 85°C.

14. The use of astacin (2) as intermediate for preparing (3R/S, 3'R/S)-astaxanthin (1a), wherein the exocyclic double bonds in (1a) have either E or E and/or Z configuration 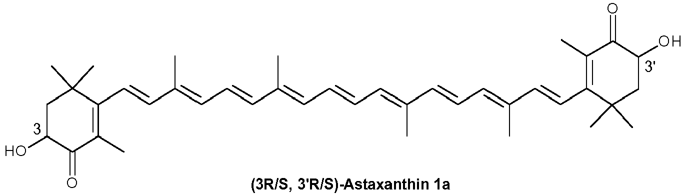 and/or (3S, 3'S)-astaxanthin (1b), wherein the exocyclic double bonds in (1b) have either E or E and/or Z configuration 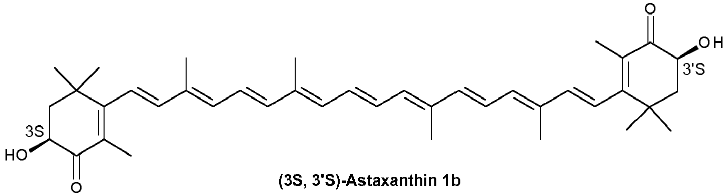 and/or (3R, 3'R)-astaxanthin (1c), wherein the exocyclic double bonds in (1c) have either E or E and/or Z configuration (1c)

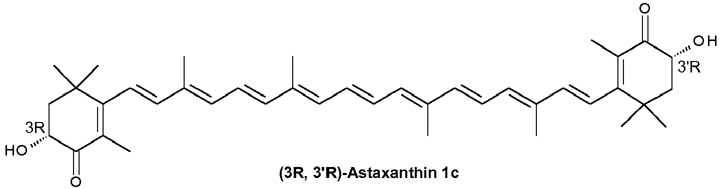

## Revendications

1. Procédé de fabrication d'astaxanthine de formule (1) 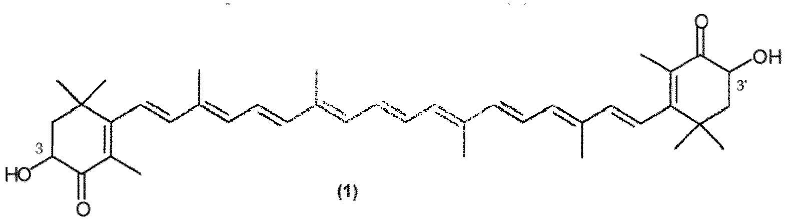 dans laquelle le centre d'asymétrie en position 3 et 3' est de configuration racémique ou respectivement (S) ou (R), et les doubles liaisons exocycliques sont de configuration E ou E et/ou Z, **caractérisé en ce que** de l'astacine de formule (2) 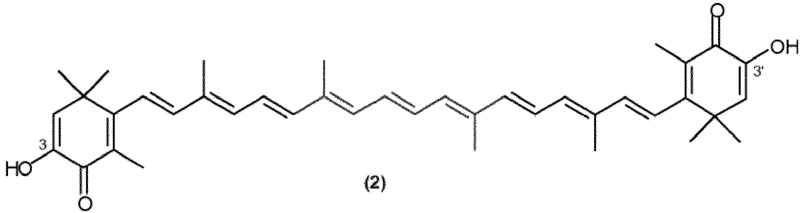 dans laquelle les doubles liaisons exocycliques sont de configuration E ou E et/ou Z, est mise en réaction de manière non stéréosélective ou de manière stéréosélective avec un réducteur,
le réducteur étant au moins un composé qui est choisi dans le groupe constitué par un alcool secondaire ; l'acide formique, les sels de l'acide formique.

2. Procédé selon la revendication 1, **caractérisé en ce que** l'astacine (2) est mise en réaction de manière non stéréosélective ou de manière stéréosélective avec le réducteur en présence d'un catalyseur de métal de transition.

3. Procédé selon la revendication 2, **caractérisé en ce que** le catalyseur de métal de transition contient un métal de transition qui est choisi dans le groupe constitué par Ti, Zr, Hf, V, Nb, Ta, Cr, Mo, W, Mn, Re, Fe, Ru, Os, Co, Rh, Ir, Ni, Pd, Pt, Cu, Ag et Au.

4. Procédé selon la revendication 2 ou 3, **caractérisé en ce que** le catalyseur de métal de transition contient au moins un ligand qui est choisi parmi les amines et/ou les phosphanes.

5. Procédé selon la revendication 4, **caractérisé en ce que** le ligand est un phosphane de formule générale (3) 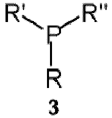 dans laquelle R, R' et R" sont choisis indépendamment les uns des autres dans le groupe constitué par au moins un des radicaux alkyle en C1-C4, phényle, aryle substitué une à trois fois par alkyle en C1-C4.

6. Procédé selon l'une quelconque des revendications 2 à 5, **caractérisé en ce que** le catalyseur de métal de transition contient au moins un ligand, qui est choisi dans le groupe constitué par H₂N-CH₂-CH₂-OH, MeHN-CH₂-CH₂-OH, H₂N-CH₂-CH₂-NH₂, TsNH-CH₂-CH₂-NH₂, TsNH-CH₂-CH₂-NH-(CH₂)ₙ-Oₘ-(CH₂)ₒ-aryle avec n = 1 à 4, m = 0 ou 1 et o = 1 à 4 et aryle = phényle ou mono-, di-, tri-alkylphényle en C1-C4, un composé optiquement actif.

7. Procédé selon l'une quelconque des revendications 2 à 5, **caractérisé en ce que** le catalyseur de métal de transition contient au moins un ligand qui est choisi dans le groupe constitué par une amine optiquement active, notamment H₂N-CHPh-CHPh-OH, H₂N-CHMe-CHPh-OH, MeHN-CHMe-CHPh-OH, TsNH-CHPh-CHPh-NH₂, (1S,2S)-N-p-toluène-sulfonyl-1,2-diphényléthylène-diamine, (1R,2R)-N-p-toluène-sulfonyl-1,2-diphényléthylène-diamine, N-[(1S,2S)-1,2-diphényl-2-(2-(4-méthylbenzyloxy)éthylamino)-éthyl]-4-méthylbenzènesulfonamide, N-[(1R,2R)-1,2-diphényl-2-(2-(4-méthylbenzyl-oxy)éthylamino)-éthyl]-4-méthylbenzène-sulfonamide, un acide aminé optiquement actif.

8. Procédé selon la revendication 4, 6 ou 7, **caractérisé en ce que** le ligand est déprotoné.

9. Procédé selon l'une quelconque des revendications 3 à 8, **caractérisé en ce que** le métal de transition est appliqué sur un support solide.

10. Procédé selon l'une quelconque des revendications 1 à 9, **caractérisé en ce que** l'astacine (2) est mise en réaction de manière non stéréosélective ou de manière stéréosélective avec un réducteur dans un milieu basique.

11. Procédé selon l'une quelconque des revendications 1 à 10, **caractérisé en ce que** l'astacine (2) est mise en réaction de manière non stéréosélective ou de manière stéréosélective avec un réducteur dans un milieu liquide, de préférence dans un milieu liquide qui est constitué par plus de 50 % en volume d'au moins un solvant organique.

12. Procédé selon la revendication 11, **caractérisé en ce que** le solvant organique contient au moins un composé qui est choisi dans le groupe constitué par le dichlorométhane, l'éther diméthylique d'éthylène glycol, l'éther diméthylique de diéthylène glycol, le tétrahydrofurane, l'acétonitrile, le carbonate d'éthylène et le carbonate de propylène.

13. Procédé selon l'une quelconque des revendications 1 à 12, **caractérisé en ce que** l'astacine (2) est mise en réaction de manière non stéréosélective ou de manière stéréosélective avec un réducteur à une température de 10 °C à 85 °C.

14. Utilisation d'astacine (2) en tant qu'intermédiaire pour la fabrication de (3R/S,3'R/S)-astaxanthine (1a), les doubles liaisons exocycliques dans (1a) étant de configuration E ou E et/ou Z 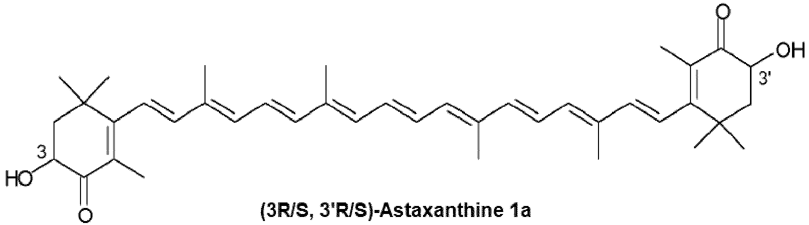 et/ou de (3S,3'S)-astaxanthine (1b), les doubles liaisons exocycliques dans (1b) étant de configuration E ou E et/ou Z 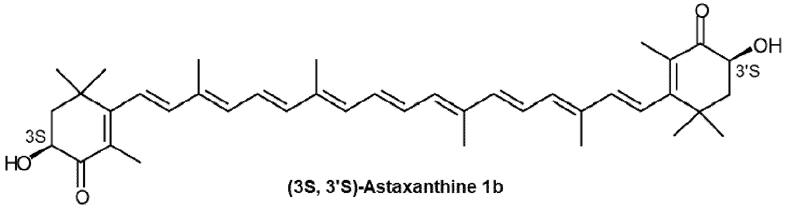 et/ou de (3R,3'R)-astaxanthine (1c), les doubles liaisons exocycliques dans (1c) étant de configuration E ou E et/ou Z (1c)
